# EUROPEAN PATENT SPECIFICATION

(11) **EP 2 359 134 B1**
(45) Date of publication and mention of the grant of the patent: **04.06.2014**
(21) Application number: 09760341.9
(22) Date of filing: 17.11.2009
(51) Int. Cl.: G01N 33/68, A61K 38/17, A61K 48/00

(54) **METHODS FOR IDENTIFYING MODULATING COMPOUNDS OF LYMPHANGIOGENESIS, MEANS THEREFORE, COMPOUNDS AND USES THEREOF**
VERFAHREN ZUR IDENTIFIZIERUNG MODULIERENDER VERBINDUNGEN DER LYMPHANGIOGENESE, MITTEL DAFÜR, VERBINDUNGEN UND VERWENDUNGEN DAVON
PROCÉDÉS PERMETTANT D'IDENTIFIER DES COMPOSÉS DE MODULATION DE LA LYMPHANGIOGENÈSE, MOYEN CORRESPONDANT, COMPOSÉS ET LEURS UTILISATIONS

(30) Priority: 17.11.2008 EP 08169305; 03.06.2009 EP 09161876
(43) Date of publication of application: 24.08.2011
(73) Proprietor: Koninklijke Nederlandse Akademie van Wetenschappen, 1011 JV Amsterdam (NL)
(72) Inventor: SCHULTE-MERKER, Stefan, NL-1217 TE Hilversum (NL); HOGAN, Benjamin, M., St Lucia, Queensland 4072 (AU)
(74) Representative: Jansen, Cornelis Marinus
(86) International application number: PCT/NL2009/050693
(87) International publication number: WO 2010/056123

(56) References cited:
- WO-A-2005/043993
- WO-A-2006/122363
- WO-A2-2005/112619

## Description

### FIELD OF THE INVENTION

The invention relates to the field of medicine. In particular the invention relates to the fields of lymphomagenesis and cancer.

### BACKGROUND OF THE INVENTION

The lymphatic vasculature plays critically important roles in inflammation, immunity, the drainage of dietary fatty acids and in cancer metastasis. Clinical evidence suggests that the dissemination of malignant tumors to regional lymph nodes via the lymphatic vessels is important in tumor metastasis and that chronic inflammation clauses lymphangiogenesis and lymphedema. In addition to VEGF-C, a potent lymphangiogenic growth factor, VEGF-A, bFGF, HGF, angiopoietin-1, IGF-1/2, and PDGF-BB, previously known as proangiogenic factors, have lymphangiogenic activity. In addition, bioactive lipid molecules, including S1P, have been reported to induce in vitro and in vivo lymphangiogenesis by stimulating the migration and differentiation of lymphatic endothelial cells via a S1P1/Gi/PLC/Ca2+ signaling pathway (Chang Min Yoon et al. Brood. 2008 August 15; 112(4): 1129-1138).

Although the molecular mechanisms regulating lymphangiogenesis are largely unclear, it has been determined that during development, regenerative growth or pathogenesis, lymphatic vessels arise from pre-existing vessels by lymphangiogenesis, a dynamic process involving the budding, migration and proliferation of lymphangioblasts, the precursors of the lymphatic vasculature.

Manipulation of lymphangiogenesis offers the opportunity for therapeutic strategies designed to inhibit or stimulate growth of lymphatic vessels in conditions such as lymphedema, cancer and infectious diseases.

In the art, such strategies have been developed based on stimulating lymphangiogenic factors such as using members of the VEGF-C family (US7150970, US6818220, WO0151075). As such, a need exists for methods and compositions for manipulating lymphangiogeneis.

### DESCRIPTION OF THE INVENTION

The invention relates in a first aspect to a method for testing whether a compound is capable of inhibiting the development of lymphatic channels or lymphangiogenesis and/or the migration of lymphangioblasts in an non-human animal, a non-human embryo or a cell culture, comprising steps of:
- contacting a compound capable of interacting with a Ccbe1 gene, a transcript thereof or a ccbe1 protein with a non-human animal, a non-human embryo or a cell culture; and
- determining whether said compound inhibits the development of lymphatic channels, lymphangiogenesis and/or migration of lymphangioblasts in said non-human animal, a non-human embryo or a cell culture.

With the term "compound" is meant any chemical compound. The compound is preferably a small molecule, an antibody or functional fragment thereof or an antisense agent.

With the term "contacting" of a compound is meant providing said compound in a sufficiently high dose such that said compound can interact with its molecular target, being the Ccbe1 gene, a transcript thereof or a Ccbe1 protein. Ccbe1 is a secreted protein, i.e. a protein that is transported to the exterior of the cell. The translated protein contains a signal peptide that is cleaved of fprior to transport to the exterior of the cell. The secreted protein is likely associated with the outside of a cell or extracellular matrix. Methods of contacting therefore may differ between molecular targets, but may also differ depending on the type of compound. Methods of providing compounds to animals and embryos are well known in the art. Compounds may be administered in any effective, convenient manner including, for instance, administration by topical, oral, anal, vaginal, intravenous, intraperitoneal, intramuscular, subcutaneous, intranasal or intradermal routes among others. A skilled person will be able to select a suitable method. Preferably, administration of a compound to an animal or an embryo is done by injection, because this results in the best uptake by target cells.

A skilled person can test any compound capable of interacting with a Ccbe1 gene, a transcript thereof or a ccbe1 protein. With the term "interacting" is meant any physical interaction wherein covalent or non-covalent binding between said compound and Ccbe1 gene, a transcript thereof or a ccbe1 protein takes place. The term "Ccbe1" is meant to refer to a protein that is the collagen and calcium binding EGF domains 1. Ccbe1 also refers to the nucleic acid (DNA or RNA) encoding the Ccbe1 protein. Representative sequences of ccbe1 can be found, for example, without limitation, in Genbank Accession Nos. NC_005117.2 (*Rattus Norvegicus*), NC_000018.8 (Homo Sapiens), NC_000084.5 (*Mus Musculus*), NC_006127.2 (*Gallus Gallus*). The Ccbe1 gene is also described in WO2006/122363 and WO2005/11269. Figure 4 depicts a human Ccbe1 nucleotide and protein sequence. Based on the nucleic acid sequence or amino acid sequence based thereon, a skilled person is able to design molecules capable of interacting with the Ccbe1 gene, a transcript thereof or a Ccbe1 protein. Of course one can select compounds that are known to interfere with Ccbe1 protein or Ccbe1 expression regulation. By testing these compounds using the method of the invention, valuable information is obtained whether these compounds are capable of inhibiting lymphangiogenesis. Antibodies directed against Ccbe1 protein are commercially available (Novus Biologicals, Mouse anti Human). Methods for selecting antibodies with affinity for a molecule are also known. A skilled person can therefore produce antibodies against Ccbe1 protein and test such antibodies using this method.

Antisense agents can also be used and are commercially available (Santa Cruz Biotechnology Inc.). Methods for producing antisense agents against a known target sequence are known in the art. A skilled person is capable of testing whether an antisense agent is capable of blocking the expression of Ccbe1 protein by providing said antisense agent to a cell and determining the expression level of Ccbe1.

With the term "animal" is meant any live non-human animal, including free-living larval and/or reproducing larval forms, but excluding fetal or embryonic forms. Said animal can be any non-human animal which has a lymphatic system. Preferably said animal is a vertebrate, because the lymphatic system of vertebrates is evolutionary more related to humans than lymphatic systems of other animals. Preferably said animal is a mammal, more preferably a rodent or primate. Preferably a non-human primate. With the term "embryo" is meant an organism in the early stages of growth and differentiation. With the term "cell culture" is meant any in vitro culture of cells outside the body. Suitable cell cultures are cell culture in which lymphangiogenesis is mimicked and include human cell cultures. Any *in vitro*-assay mimicking lymphangiogenesis can be used. Such assays are known in the art. For example, an in-vitro tube assay using lymphatic endothelial cells (LEC) and an invasion assay using LEC are described (Nakamura Cancer Sci (95);No.1;p.25-31(2004). Said animal can also include humans, while the term "non-human animal" refers to any non-human animal including mammals and preferably a rodent or primate.

In a preferred embodiment, such in-vitro assay comprises an in vitro migration test. Preferably such in vitro migration test comprises placing cells (preferably LECs) into a chamber (Boyden chamber) that allows cells to move through a membrane (the bottom of the chamber) in response to a stimulus.

Preferably, such in vitro migration test is based on a chamber of two medium-filled compartments separated by a microporous membrane. Typically, cells are placed in the upper compartment and are allowed to migrate through the pores of the membrane into the lower compartment, in which an agent is present. Preferably, after an appropriate incubation time, the membrane between the two compartments is fixed and stained, and the number of cells that have migrated to the lower side of the membrane is determined. Such in vitro migration test may also comprise a filter membrane migration assay or a trans-well migration assay. A number of different Boyden chamber devices are available commercially.

Preferably, such in vitro migration test comprises placing LECs into a Boyden chamber, induce migration with VEGFC, and determine whether knockdown of ccbe1 affects migration. In another preferred embodiment, such in vitro migration test comprises placing LECs on one side of the membrane, and provide Ccbe1 protein on the other side of the membrane preferably in the form of cells induced to express Ccbe1). Preferably, migration or other behaviour of LECs is compared in the presence or absence of Ccbe1 protein.

It is of course possible to add other cell types, such as tumour cells to such assay. Such assay with additional tumour cells can be used to screen compounds capable of inhibiting lymphangiogenesis in tumours. In a preferred embodiment said culture comprises a co-culture of two different types of cells. Preferably, at least one of said cell types comprises endothelial cells, preferably LEC cells. In a preferred embodiment, said other type of cells are cells expressing ccbe1. In a preferred embodiment, said endothelial cells and said ccbe1 are compatible, usually they are derived from the same animal species. Said two different types of cells are preferably but not necessarily from the same animal species. In one embodiment said other type of cells are CHO cells. Preferably said CHO cells comprise an expression cassette for the expression of a primate ccde1. In a preferred embodiment said endothelial cells are primate endothelial cells. Preferably said primate cells are human cells. Preferably said primate ccbe1 is a human ccbe1.

With the term "lymphatic channel" is meant a vascular duct that carries lymph which is eventually added to the venous blood circulation. With the term "lymphangiogenesis" is meant the process of the formation of lymphatic channels. With the term "lymphangioblast" is meant a precursor cell capable of differentiation into a cell present in a lymphatic channel. Lymphangioblasts can be detected by the presence of membrane bound marker, such as VEGF-C; VEGFR-3 or Prox1, or by the expression of stabilin1, tie2, lyve1 and/or fli1. With the term "migration"of a lymphangioblast is meant the process starting with budding of a lymphangioblast from a vein and migrating of said lymphangioblast into mesordermal tissue and ends when said lymphangioblast starts to contribute to lymphangiogenesis. Methods to detect migration of lymphangatic endothelial cells are known in the art.

Methods for detecting lymphatic channels and cells in an animal are known to a skilled person. Lymphatic channels and lymphatic cells can be visualized by imaging. Detection can be done by using markers or labels specific for lymphatic channels, lymphatic cells or lymphatic endothelial cells. For example, antibodies directed against specific antigens can be used. A preferred method to determine whether the development of lymphatic channels or lymphangiogenesis is inhibited is by determining the presence of a thoracic duct or part thereof in a non-human animal or a non-human embryo. In a non-human mammal it is preferred to determine whether the development of lymphatic channels or lymphangiogenesis is inhibited in the ear. Inhibition can be measured by measuring swelling of the ear due to inadequate drainage via the lymphe.

The absence or reduction in the number of lymphatic channels, cells or lymphatic endothelial cells in said animal, embryo or cell culture is indicative for inhibition of development of lymphatic channels or lymphangiogenesis. With the term "inhibiting the development of lymphatic channels and/or lymphangiogenesis" is meant that the volume or the number of lymphatic channels or the number of cells involved in the process of lymphangiogenesis is significantly lowered in said animal, embryo or cell culture, compared to a control which has not been provided with said compound. Inhibition of the migration of a lymphangioblast is defined as an interference with the migration of a lymphangioblast, with a result that said lymphangioblast does not contribute to lymphangiogenesis. Preferably, inhibition between 70-100% is achieved. More preferably, between 80 and 100% is achieved. Even more preferably between 90 and 100%. Even more preferably between 95 and 100%. Most preferably said inhibition is between 97 and 100%.

In a preferred embodiment, said animal or said embryo is transparent, at least for the duration of the experiment. An advantage thereof is that this facilitates imaging of the development of lymphatic channels or lymphangiogenesis and/or the migration of lymphangioblasts.

In another aspect, the invention relates to a method for testing whether a compound is capable of inhibiting the development of lymphatic channels or lymphangiogenesis and/or the migration of lymphangioblasts in a non-human animal, a non-human embryo or a cell culture, comprising the steps of administering a compound to a non-human animal, a non-human embryo or a cell culture, wherein said non-human animal, said non-human embryo, or cell culture does not express the Ccbe1 protein at a functional level; inducing expression of Ccbe1 protein in cells of said non-human-animal, said non-human embryo, or said cell culture; and determining whether said compound inhibits the development of lymphatic channels, lymphangiogenesis and/or migration of lymphangioblasts in said non-human animal, a non-human embryo or a cell culture.

An advantage of this method is that any compound, without prior knowledge of any interaction with Ccbe1 gene, transcript or protein can be tested. With "expressing at a functional level" is meant that expression of the Ccbe1 protein results in development of lymphatic channels, lymphangiogenesis and/or migration of lymphangioblasts in said non-human animal, a non-human embryo or a cell culture.

In some embodiments, the cells that do not express the Ccbe1 protein at a functional level in a non-human animal or embryo are cells that in a wild-type organism do express the protein at a functional level.

Inhibition of the function and/or expression of Ccbe1 protein in a system for development of lymphatic channels, lymphangiogenesis and/or migration of lymphangioblasts, inhibits this development, genesis and/or migration. Preferably, expression is reduced between 70-100%. More preferably, between 80 and 100%, even more preferably between 90 and 100%, even more preferably between 95 and 100%. Most preferably said expression is reduced between 97 and 100% compared to normal levels. There are several methods known to block the functional expression of a gene. It is possible to block the expression of the Ccbe1 gene by providing an animal with antisense agents capable of binding to nucleotides of the Ccbe1 gene or gene product. Based on the sequence of the Ccbe1 gene, a skilled person will be able to design an antisense agent that is capable of binding to said gene. Preferred examples of are described in the example section. Expression of the Ccbe1 gene can also be substantially blocked by knocking out or mutating the ccbe1 gene such that it is rendered dysfunctional. A person skilled in the art will know how to make knockout animals. Examples of knockout procedures using cre/lox recombination systems can be found in Lakso et al. (1992) Proc. Natal. Acad. Sci. 89: 6232-36 and Pichel et al. (1993) Oncogene 8: 3333-42. A preferred method for generating mice heterozygous for a Plox-site flanked version of the ccbe1 gene targets exons 4 and 5. These mice can then be crossed with mice expressing cre-recombinase under the control of an inducible promoter or a tissue-specific promoter, and resulting homozygous mutant mice will be lacking the gene activity in all cells that express Cre-recombinase.

A skilled person is familiar with several methods to induce expression of a protein. If the expression of Ccbe1 is suppressed, for example, under influence of an inducible repressor, the repression can simply be reversed by removing the substrate which binds to the repressor. Another method for inducing expression of Ccbe1 is by injection of mRNA encoding wild-type Ccbe1 into said animal. An example of this method is provided in Example 1. A preferred method for providing cells with the capacity to express Ccbe1 protein is by means of a gene delivery vehicle comprising an expression cassette for expression of a ccbe1 coding region. In a preferred embodiment, said gene delivery vehicle comprises a adenoviral vector, an adeno-associated viral vector or a lentiviral viral vector. Preferably said gene delivery vehicle comprises a gene encoding a ccbe1 protein.

In a preferred embodiment, said non-human animal or said non-human embryo is genetically modified, wherein the modification comprises the transgenic expression of stabilin1, tie2, lyve1 and/or fli1 fused to a GFP or a derivative thereof. With the term "derivative" of GFP is meant any mutated form of GFP resulting in an improved functionality of GFP, for example blue fluorescent protein (EBFP, EBFP2, Azurite, mKalama1) cyan fluorescent protein (ECFP, Cerulean, CyPet), yellow fluorescent protein derivatives (YFP, Citrine, Venus, YPet) and BFP derivatives.

The advantage thereof is that these markers facilitate the imaging of veins, and are useful when following migration of lymphangioblasts. Methods to produce animals with GFP constructs are known in the art. Examples of animals expressing tie2, lyve1 or fli1 GFP are also known in the art.

In another aspect, the disclosure relates to the use of a genetically modified non-human animal, preferably a fish wherein a ccbe1 gene is knocked out. As used herein, a "knock-out" refers to a gene that has been modified, by, e.g., chemical mutagenesis or homologous recombination, to reduce the function and/or quantity of the encoded protein. A knock-out may be generated, e.g., by removing one or more exons or by introducing a premature stop codon and may result in a null, hypomorphic, or neomorphic allele.

In some embodiments, the non-human animal is Drosophila or a mouse. The generation of knock-out mutations in these species is well within the purview of one skilled in the art. Preferably, the genetically modified animal is a fish. Methods for knocking out ccbe1 are provided in the examples. The advantage of said genetically modified fish is that is can advantageously used in the above described methods. More preferred is a fish, wherein cells of said fish further expresses stabilin1, tie2, lyve1 and/or fli1 fused to a GFP or a derivative thereof. In another aspect, the invention relates to a genetically modified non-human animal, preferably a fish wherein stabilin1, tie2, lyve1 and/or fli1 fused to a GFP or a derivative thereof is expressed. More preferably, said fish is a zebrafish, *Danio Rerio*. The advantage of a zebrafish is that they are easy to grow and culture.

In another aspect, the disclosure relates to the use of a cell expressing stabilin1, tie2, lyve1 and/or fli1 fused to a GFP or a derivative thereof. Said cell can be used in the methods by transplanting said cells in a non-human animal and determine whether said cells contribute to lymphangiogenesis. Said cells can of course also be used in cell culture. The advantage thereof is that said cells can easily be visualized. Said cell can be used as in *in vitro* or *in-vivo*-assays for testing compounds capable of influencing lymphangiogenesis. Any *in vitro*-assay mimicking lympangiogenesis can be used. Such assays are known in the art. For example, an in-vitro tube assay using LEC and an invasion assay using LEC are described (Nakamura et al., Cancer Sci (95);No.1;p.25-31(2004). Methods for knocking out ccbe1 are provided in the example. A skilled person will therefore be able to produce LECs from lymphangiomas (described in Mancardi et al. Exp Cell Res 1999; 246:368-75) derived from Ccbe1 null .mice. A skilled person is able to make Ccbe1 null mice. A skilled person will also be able to use LEC that do not express Ccbe1. For co-cultures it is preferred to use endothelial cells that do not express Ccbe1. These can be obtained from Ccbe1 null animals, or from animals that have the capability to express Ccbe1. Endothelial cells, preferably LEC, do not express Ccbe1 themselves but cells adjacent to the LECs express Ccbe1. Preferably co-cultures are used wherein endothelial cells are co-cultured with cells expressing Ccbe1.

An example of an in vitro growth assay is given in Nakamura et al. Cancer Sci (95);No.1 (2004), p 26. Above mentioned in vitro assays can of course also be used to determine whether a compound is capable of inhibiting the stimulation of lymphangiogenesis.

In another aspect, the disclosure relates to use of a cell in a method to determine whether a compound is capable of influencing lymphangiogenesis.

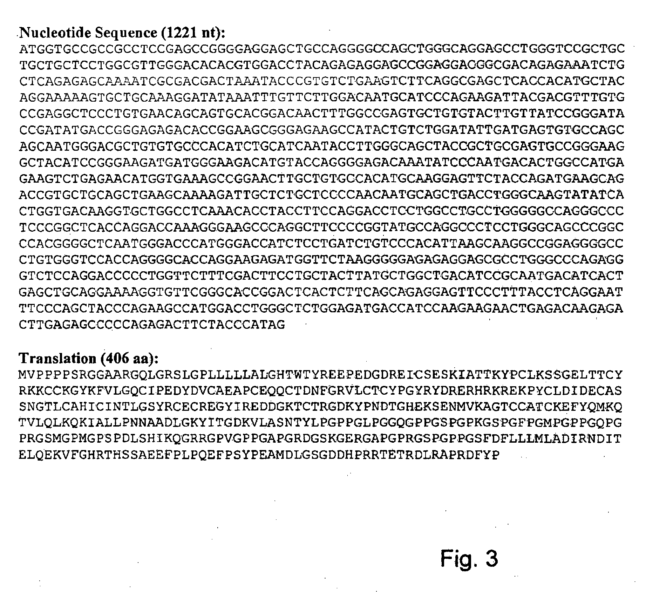

In another aspect, the disclosure relates to an antisense agent comprising the base sequence of a gene encoding Ccbe1. Preferably said genre is a human Ccbe1 gene. A preferred human ccbe1 sequence is depicted in figure 3. An advantage of said antisense agent is that it can be used to functionally block Ccbe1 expression. An "antisense agent" refers to an oligonucleotide that interacts with complementary strands of nucleic acids, modifying expression of genes. An oligonucleotide is a short segment of nucleotides, typically with between 15-40 fewer bases. Preferably said oligonucleotide comprises between 15-30 bases. More preferably said oligonucleotide comprises between 20-30 bases. For morpholino compounds it is preferred that said oligonucleotide comprises between 18-25 consecutive bases complementary to the target RNA. Many forms of antisense molecules are known in the art and can be categorized into enzyme-dependent antisense or steric blocking antisense. Any antisense molecule capable of substantially blocking the expression of Ccbe1 protein can be used. Examples of suitable antisense agents comprise, but are not limited to: a RNAi/siRNA (Caplen et al. Proc Natl Acad Sci U S A. 2001 Aug 14;98(17):9742-7), a morpholino oligomer (Summerton Biochim Biophys Acta. 1999 Dec 10;1489(1):141-58), a peptide nucleic acid (Hanvey et al. Science. 1992 Nov 27;258(5087):1481-5), a 2'-O-aliyl or 2'-O-alkyl modified oligonucleotide, a N3'.fwdarw.P5' phosphoramidate oligonucleotide (Gryaznov Biochim Biophys Acta. 1999 Dec 10;1489(1):131-40), a C-5-propyne pyrimidine-modified oligonucleotide or an "RNAse-inactive" oligonucleotide. A morpholino is assembled from four different morpholino subunits, each of which contains one of the four genetic bases linked to a 6-membered morpholine ring. Eighteen to 25 subunits of these four subunit types are joined in a specific order by non-ionic phosphorodiamidate intersubunit linkages to give a morpholino. A "N3'.fwdarw.P5' phosphoramidate" oligonucleotide is one in which the 3'-oxygen of the 2'-deoxyribose is replaced by a 3'-amine. A "2'-O-allyl (or alkyl) modified oligonucleotide" is an oligoribonucleotide in which the 2' hydroxylis converted to an allyl or alkyl ether. The alkyl ether is typically a methyl ether. "C-5-propyne pyrimidine-modified oligonucleotide" is an oligonucleotide in which the C-5 methyl group of thymidine bases and/or the C-5 hydrogen of cytidine bases has been replaced with a propyne group. In a "peptide nucleic acid", the deoxyribose phosphate units of an oligonucleotide backbone are replaced with polyamide linkages. A "RNAse-inactive" oligonucleotide or oligonucleotide analog is one which acts via an RNase-independent mechanism, unlike RNAse-active oligonucleotides, such as phosphorothioates. They are believed to function by sterically blocking target RNA formation, nucleocytoplasmic transport or translation, and are thus also referred to as "steric blockers". This class includes, for example, methylphosphonates, morpholino oligonucleotides, as described herein, peptide nucleic acids (PNA's), 2'-O-allyl or 2'-O-alkyl modified oligonucleotides, and N3'.fwdarw.P5' phosphoramidates.

In a preferred embodiment said anti-sense agent comprises a sequence 5'-CGGGTAGATCATTTCAGACACTCTG-3', 5'-ACAGCACAGCACTTTACCTGTCTAC-3' or 5'-ATTAGCATAGGGAACTTACTTTCG-3'. These three oligonucleotides are directed against zebrafish ccbe1 RNA. It is preferred that an oligonucleotide of the invention is directed toward the corresponding region of a human ccbe1 RNA. In a particularly preferred embodiment said anti-sense agent is used to inhibit the development of lymphatic channels or lymphangiogenesis and/or the migration of lymphangioblasts.

In another aspect, the disclosure relates to an antisense agent having between 15-40 nucleobases in length comprising at least between 15-40 consecutive bases complementary to and able to hybridize with the nucleotide sequence of figure 3.

In another aspect, the disclosure describes a compound obtainable by the method according to the invention. An advantage of said compound is that it inhibits lymphangiogenesis, the development of lymphatic channels or the migration of lymphangioblasts.

In another aspect, the disclosure relates to an antibody or a functional fragment thereof capable of binding to a ccbe1 protein and capable of inhibiting lymphangiogenesis. With the term "antibody" is meant any immunoglobulin. Any immunoglobulin produced by any means or derived from any animal capable thereof can be used. With the term "functional fragment" of an antibody is meant a functional fragment of said antibody that comprises an antigen binding site. For example, Fab fragments can be used. It is essential that said antibody is capable of binding the ccbe1 protein. Not all antibodies capable of binding to the Ccbe1 protein may be capable of inhibiting the development of lymphatic channels, lymphangiogenesis and/or the migration of lymphatic endothelial cells in an animal. Without being bound by theory, it is believed that Ccbe1 protein is a secreted regulator of lymphangiogenesis.

In another aspect, the disclosure relates to a pharmaceutical composition, comprising an antisense agent according to the invention, an expression system according to the invention, a compound according to the invention, and/or an antibody according to the invention and a suitable pharmaceutical carrier or an adjuvant. Suitable pharmaceutical carriers or adjuvants for antibodies are well known in the art. A pharmaceutical carrier can be any compatible, non-toxic substance suitable for delivery of the compound to the patient, Sterile water, alcohol, fats, waxes, and inert solids may be included in the carrier. Pharmaceutically accepted adjuvants (buffering agents, dispersing agent) may also be incorporated into the pharmaceutical composition. The invention also provides the use of said composition in therapy.

In another aspect, the disclosure relates to the use of an antisense agent according to the invention, a compound according to the invention, and/or an antibody according to the invention for the production of a medicament.

In another aspect, the disclosure describes the use of an antisense agent according to the invention, a compound according to the invention, and/or an antibody according to the invention for inhibiting the development of lymphatic channels or lymphangiogenesis and/or the migration of lymphangioblasts.

In another aspect, the disclosure relates to the use according to the invention for the treatment of cancer. In particular, the invention relates to the use of an antisense agent according to the invention, a compound according to the invention, and/or an antibody according to the invention. The advantage of said use is that it is capable of preventing tumour growth in an animal. Without being bound by theory, it is believed that tumour growth is prevented because tumours require lymphatic vasculature.

In another aspect, the disclosure provides a method for treating an individual afflicted with cancer, comprising providing said individual in need thereof with a therapeutically effective amount of a Ccbe1 inhibitor. In another aspect, the invention provides a method for treating an individual afflicted with lymphedema, comprising providing said individual in need thereof with a therapeutically effective amount of a Ccbe1 inhibitor. In some embodiments, the Ccbe1 inhibitor is selected from an antisense agent according to the invention, a compound according to the invention, and/or an antibody according to the invention. In some embodiments, the Ccbe1 inhibitor is formulated in a pharmaceutical composition suitable for human administration.

In another aspect, the disclosure relates to the use of an expression system according to the invention for the production of a medicament for the treatment of lymphedema. Lymphedema is a condition of localized fluid retention caused by a compromised lymphatic system. By providing an expression system capable of stimulating lymphangiogenesis, the effects of lymphedema can be effectively treated.

In another aspect, the disclosure describes a method for influencing the development of lymphatic channels, lymphangiogenesis and/or the migration of lymphangioblasts in an animal comprising administration of an effective amount of a compound according to the invention, an antibody according to the invention, an antisense agent according to the invention or an expression system according to the invention to an animal. Any animal, including humans can be treated.

More preferred is a method wherein said influencing comprises inhibiting the development of lymphatic channels, lymphangiogenesis and/or the migration of lymphangioblasts in an animal.

Further provided is a method wherein said influencing comprises stimulating the development of lymphatic channels, lymphangiogenesis and/or the migration of lymphangioblasts in an animal.

In a further aspect, the disclosure describes a method of determining whether an individual is a carrier, or is suffering from, or at risk of suffering from, a lymph vessel disorder, comprising providing a sample from said individual; determining the presence of an alteration in the sequence of the Ccbe1 gene, and determining that the individual is a carrier, or is suffering from, or at risk of suffering from, the lymph vessel disorder if said alteration is present.

Said alteration in the sequence of the Ccbe1 gene results in a functional silencing of the Ccbe1 gene. With the term functional silencing is meant that the activity of the Ccbe1 protein is reduced, compared to the protein product of a Ccbe1 gene without said alteration. A reduction of activity may result from a reduction of the expression of the gene. For example, an alteration in the enhancer or promoter of the Ccbe1 gene, or an alteration of the mRNA leader sequence or trailer sequence resulting in a reduced stability of the mRNA, will lead to a reduction of the expression of the gene. Thus, in a method of the invention, the non-coding sequences of the Ccbe1 gene are determined, for example by sequence analysis, to identify one or more alterations that reduce the expression from the Ccbe1 gene.

In a preferred embodiment, an alteration in the sequence of the Ccbe1 gene comprises an alteration of the coding sequence of the Ccbe1 protein. Said alteration of the coding sequence results, for example, in reduced stability of the protein, reduced calcium-binding activity, reduced collagen-binding activity, or reduced binding of the protein to its receptor. Preferred alterations for the method of the invention are amino acid alterations comprising amino acids C75, C102, C174, L229 and/or G327. More preferred are alterations of amino acid C75 to S75 (C75S), C102S, C174R, L229fsX8 and/or G327R.

Methods for determining alterations in the coding region of the Ccbe1 gene are available in the art. For example, the amino acid sequence of the protein can be determined by mass spectrometry, for example Matrix Assisted Laser Desorption Ionisation-Time of Flight (MALDI-TOF)) or Edman degradation reaction. Alternatively, antibodies that specifically identify an epitope of the Ccbe1 protein can be used to determine the presence of an alteration of the coding sequence of the Ccbe1 protein. Further preferred methods comprise analysis of the nucleic acid sequence of the Ccbe1 mRNA, or a copy DNA of the mRNA, and analysis of the nucleotide sequence of one or more of the coding exons of the Ccbe1 gene. Methods to analyse the nucleic acid sequence of a nucleic acid are known in the art and comprise, for example, dye-termination sequencing, pyrosequencing, sequencing by ligation, and Sanger sequencing. An amplification step, such as for example a polymerase chain reaction, may precede the analysis of the nucleotide sequence of one or more of the coding exons of the Ccbe1 gene.

The use of antibodies may also be employed to determine if an alteration in the Ccbe1 gene is present. For example, mutations in the Ccbe1 gene that lead to reduced expression or alteration in the length of the encoded protein can be determined by the reduction in signal or altered band size in a Western Blot. Disclosed also are antibodies that specifically recognize an epitope selected from C75S, C102S, C174R, L229fsX8 and/or G327R of Ccbe1, which can be used, e.g., to detect the specific mutations in an immunoassay. In one embodiment, the invention provides a method of determining whether an individual is a carrier, or is suffering from, or at risk of suffering from, a lymph vessel disorder, comprising providing a sample from said subject; performing an immunoassay to determine the presence of an alteration in the sequence of the Ccbe1 gene, and determining that the individual is a carrier, or is suffering from, or at risk of suffering from, the lymph vessel disorder if said alteration is present.

With the term a sample is meant any sample comprising cells that can be obtained from an individual, preferably a human. Said sample preferably comprises blood, stool, or other body fluids comprising cells from the individual. A preferred sample comprises blood cells from the individual. Protein, or nucleic acid such as DNA and./or RNA, can be substantially fractionized and isolated from the sample as is known to a skilled person. The presence of an alteration in the sequence of the Ccbe1 gene is determined in the protein fraction, or in the nucleic acid fraction, as will be clear to a person skilled in the art.

A method described herein allows determining whether an individual is a carrier of a lymph vessel disorder, or whether the individual is suffering from, at risk of suffering from, a lymph vessel disorder. If an alteration in the sequence of the Ccbe1 gene is present on one copy of the Ccbe1 gene, the individual is heterozygous for the alteration, and is not likely to suffer from the lymph vessel disorder. Said heterozygous individual is a carrier of the lymph vessel disorder, meaning that the individual can transmit the disorder to the offspring of the individual. The method thus allows determining whether an individual is a carrier of a lymph vessel disorder who is at risk of transmitting the disorder to the progeny of the individual. If an alteration in the sequence of the Ccbe1 gene is present on both copies of the Ccbe1 gene, said individual is suffering from or at risk of suffering from said lymph vessel disorder.

Said lymph vessel disorder preferably is a congenital lymph vessel disorder resulting in lymphedema. Congenital lymphedema results from having insufficient lymphatic vessels that they cannot handle all the lymph. Lymphedema almost always affects the legs. Women are much more likely than men to have congenital lymphedema. Rarely, the swelling is obvious at birth. More often, the swelling appears later in life, as the volume of lymph increases and overwhelms the small number of lymph vessels. The swelling often starts gradually in one or both legs. A preferred congenital lymph vessel disorder for use of a method of the invention is selected from Meige, Nonne-Milroy and Hennekam syndrome. A most preferred lymph vessel disorder is Hennekam syndrome.

In yet a further aspect, disclosed is a composition comprising Ccbe1, or comprising a nucleic acid encoding Ccbe1, as a medicament. A medicament comprising Ccbe1 or comprising a nucleic acid encoding Ccbe1 can be used in the treatment of a congenital or acquired lymph vessel disorder. A preferred medicament comprises human Ccbe1, or comprises a nucleic acid encoding human Ccbe1.

In some embodiments, the human Ccbe1 protein provided in the composition, medicament, or methods disclosed herein is at least 70, 80, 90, 95, 98, or 100% identical to the human Ccbe1 protein as depicted in Figure 3.

The comparison of sequences and determination of percent identity and similarity between two sequences is well-known in the art and can be accomplished using a mathematical algorithm. (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). When a position in a first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

In one embodiment, said medicament comprises a full length human Ccbe1 protein or an active part or derivative thereof. With the term active part is meant a part of the protein that is able and sufficient to induce lymph vessel formation. With the term derivative is meant a modification of the full length protein or active part that, for example, stabilizes the protein or part thereof.

In some embodiments the human Ccbe1 protein is a functional fragment or functional derivative of the full-length protein. Functional fragments and derivatives are capable of inducing lymph vessel formation. Functional derivatives encompass full-length proteins and protein fragments of Ccbe1 comprising modifications, such as, e.g., stabilizing modifications. Ccbe1 proteins may also include various tags, such as His, Myc, etc, as well as additional heterologous sequences.

In a further embodiment, the medicament comprises a nucleic acid encoding full length Ccbe1 or a functional part thereof. In one embodiment, said nucleic acid is a naked nucleic acid molecule further comprising means for expression of the Ccbe1 protein, such as, for example, an enhancer, a promoter, and a termination signal. In a preferred embodiment, said medicament further comprises means for transducing the nucleic acid into a cell. Said means preferably comprise a virus particle encompassing the nucleic acid. A preferred virus particle is selected from an adenovirus particle, a retrovirus particle or a adeno-associated virus particle.

Said medicament for the treatment of a lymph vessel disorder selected from a congenital lymph vessel disorder or an acquired lymph vessel disorder. An acquired lymph vessel disorder typically appears after major surgical treatment, especially after cancer treatment in which lymph nodes and lymphatic vessels are removed or treated with radiation. For example, the arm tends to swell after removal of a cancerous breast and lymph nodes in the armpit. Scarring of lymphatic vessels from repeated infection also may cause lymphedema, which can be treated with a medicament according to the invention.

A preferred medicament comprises a therapeutically active amount of Ccbe1, or of a nucleic acid encoding full length Ccbe1, and one or more pharmacological accepted carriers or excipients. The medicament is administered in a pharmaceutical formulation such as in a liquid carrier for injection, or in capsule form for ingestion. Administration of a medicament to humans can be by any technique capable of introducing the ingredients including oral, intravenous, intramuscular, intradermal, and subcutaneous administration. Other therapeutic agents may also be present in the formulation.

In some embodiments, disclosed are Ccbe1 protein and/or Ccbe1 nucleic acid for use in treating a lymph vessel disorder as described herein.

In one aspect, disclosed are methods for treating a subject afflicted with a lymph vessel disorder comprising administering to a subject in need thereof a therapeutically effective amount of a medicament described herein, in particular a composition comprising Ccbe1 protein or a nucleic acid encoding encoding Ccbe1. In preferred embodiments, the subject afflicted with the lymph vessel disorder has one or more mutations in the ccbe1 gene. A preferred lymph vessel disorder for use of a method of the invention is selected from Meige, Nonne-Milroy and Hennekam syndrome. A most preferred lymph vessel disorder is Hennekam syndrome.

The invention is further explained in the following examples. These examples do not limit the scope of the invention, but merely serve to clarify the invention.

### BRIEF DESCRIPTION OF THE DRAWINGS

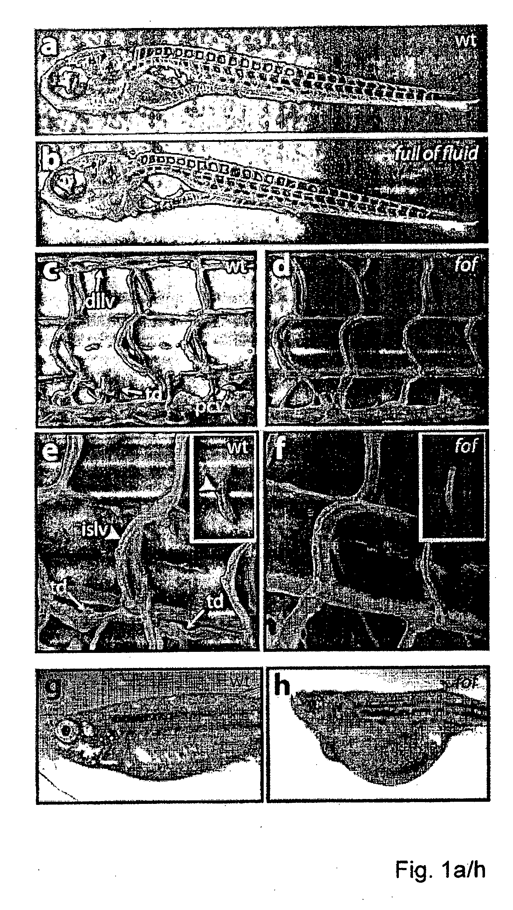

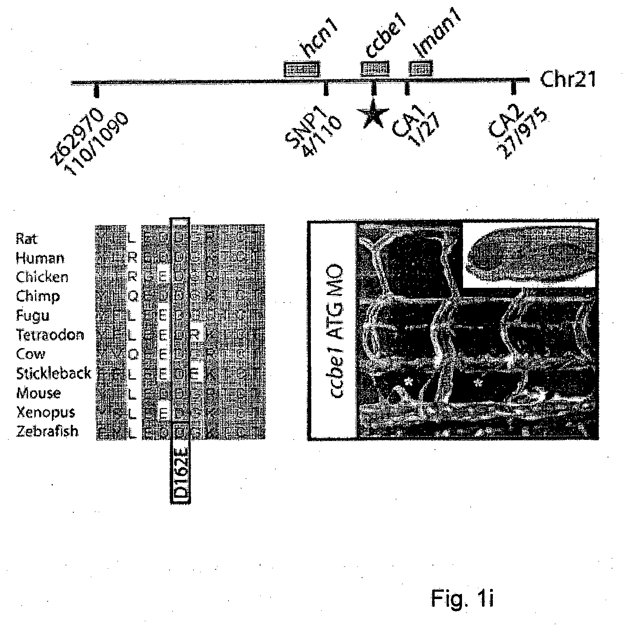

**Figure 1**: *full of fluid* mutants lack trunk lymphatic vessels.
   **a** and **b**. Overall patterning of blood vessels (*fli1:GFP,* 5dpf) in wildtype siblings (**a**) and *fof* mutants (**b**).
   **c-f**. Angiographies in *fli1:GFP* embryos at 5dpf reveal that thoracic duct (arrows in **c** and **e**), intersegmental lymphatic vessels (ISLVs) (arrowheads and inset in **e**) and dorsal longitudinal lymphatic vessel (DLLV) (arrowhead in **c**) are devoid of blood flow. *fof* mutants lack all trunk lymphatic vessels (**d** and **f**).
   **g** and **h**. *fof* mutants (**J**) that survive to 36dpf (n=3/28, see Supplementary Fig. 1)
      display severe edema
   **.i. Upper**- Meiotic map of the *fof* locus. Recombinant events for each polymorphic
      marker are depicted in red (proximal) and blue (distal). Recombinants for SNP1 and
      CA1 exclude neighboring genes *lman1* and *hcn1.*
   **Lower left**- Multiple sequence alignment of Ccbe1 proteins demonstrating the conservation of zebrafish D162.
   **Lower right**- *ccbe1* ATG morpholino phenocopies *fof* in morphologically normal (inset) embryos (n=134/136 (splice MO n=20/59)). Asterix=absence of thoracic duct.

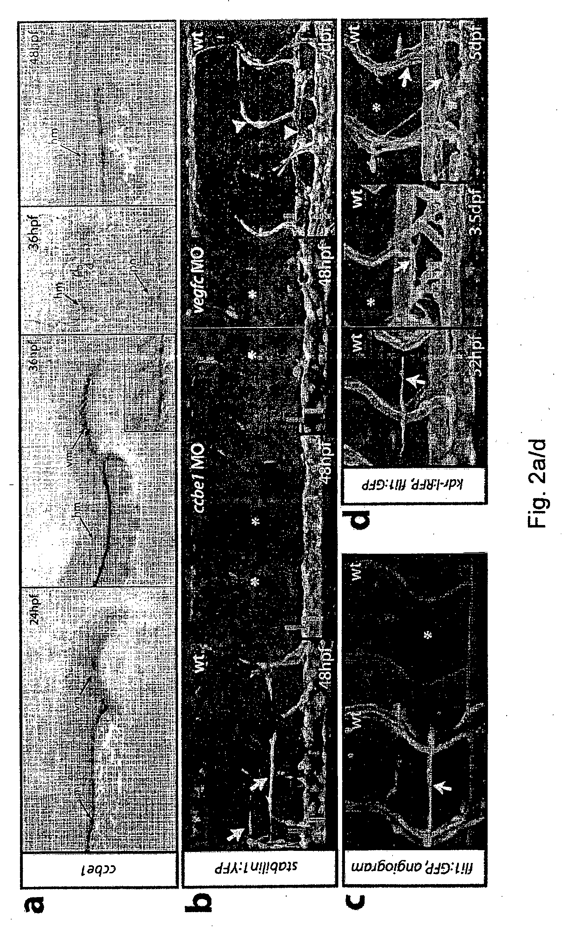

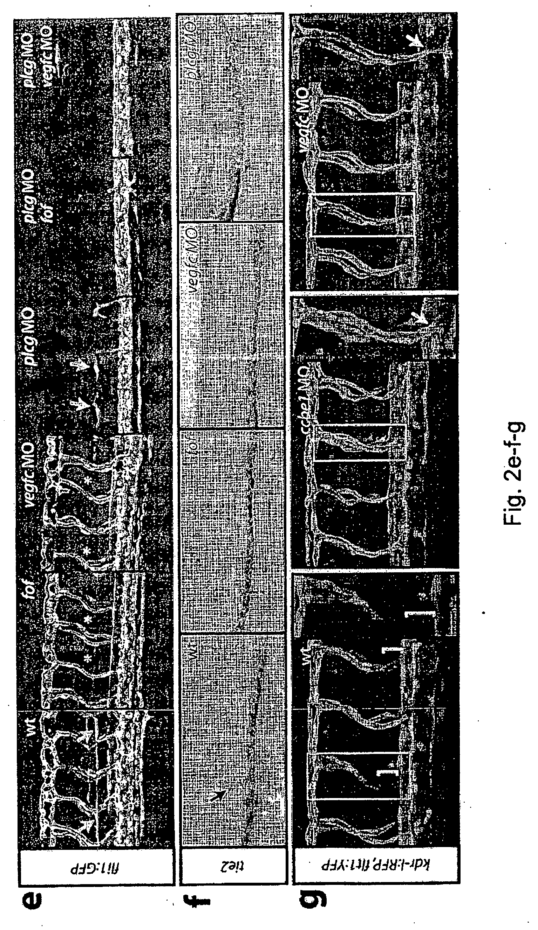

**Figure 2**: *ccbe1* and *vegfc* are required for lymphangioblast budding and angiogenic sprouting from venous endothelium.
   *a. ccbe1* trunk expression is restricted to ventral mesenchyme (vm) and pronephros (pn) at 24 hpf. At 36hpf, expression extends along somitic boundaries (inset) and the horizontal myoseptum (hm) (bilateral in section). *ccbe1* trunk expression is restricted to the horizontal myoseptum at 48hpf when lymphangioblasts populate this region.
   **b**. *stabilinl: YFP* expression marks the PCV (blue bars=PCV, red bars=DA) and
      derived lymphangioblasts (arrows indicate bilateral cells) at 48hpf, and the lymphatic vasculature (arrowheads) and PCV at 7dpf, with progressively weakening arterial expression. *stabilin1* expressing cells fail to bud in *ccbe1* (n=21/22) and *vegfc* morphants (n=29/31) (48hpf).
   c. Endothelial cells in the region of the horizontal myoseptum express *fli1* (arrow) but
      do not sustain blood flow (asterix, angiogram in red) (52hpf).
   **d**. *fli1* positive lymphangioblasts in the region of the horizontal myospetum are migratory and contribute to the lymphatic vasculature as visualized in *kdr-l:RFP*, *fli1:GFP* double transgenic animals (arrows indicate lymphangioblasts (52hpf and 3.5dpf) and lymphatic vessels (5dpf), asterix indicates absence of parachordal endothelial cells) (see also Supplementary Movie 5).
   **e**. Lymphangioblasts (*fli1:GFP*) fail to bud and migrate in *fof* mutants (n=12/12), *vegfc*
      morphants (n=16/16), *fof*/*plcg* double morphants (n=28/28) and *vegfc*/*plcg* double
      morphants (n=74/83) but not in *plcg* single morphants (n=21/21) at 48hpf. Arrows indicate lymphangioblasts, asterix indicate their absence.
   *f. tie2* is expressed in PCV sprouts in wildtype embryos which are absent in *fof*
      mutants (n=35/35), *vegfc* morphants (n=57/64) or *plcg* morphants (n=55/55) at 48hpf.
   **g**. In wildtype embryos, venous sprouts are *flt1: **YFP*** negative at 48 hpf in *kdr-l:RFP, flt1:YFP* double transgenic embryos. In *ccbe1* (n=20/20) and *vegfc* morphants (n=22/25), venous sprouts are absent and in their absence a functional circulation is established due to increased ISV connections with the dorsal aorta. Bracket indicates venous derived sprouts, arrows indicate arterial connections.
**Figure 3**: Sequence of a human ccbe1 coding sequence and protein.
**Figure 4**: Survival and morphology of *full of fluid* mutants.
   **a**. Daily survival curve of wildtype population (n=28) of *fof* siblings selected for the presence of the thoracic duct at 6dpf (blue diamonds) compared with mutant population (n=28) selected for lack of thoracic duct at 6dpf (pink squares).
   **b**. Fluid accumulation in the intestine (arrow in left panel) and around the eyes (arrows in **right panel**) in mutants (lower) compared with wildtype siblings (upper) at 6dpf.
   **c** and **d**. Morphology of 36dpf sibling (**c**) and mutant (**d**) fish (images from Fig. 1) corresponding to endpoint of two plotted populations.

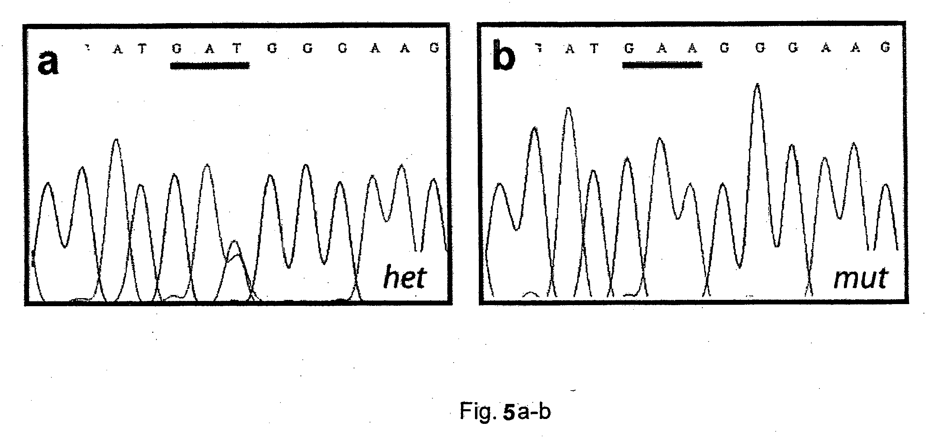

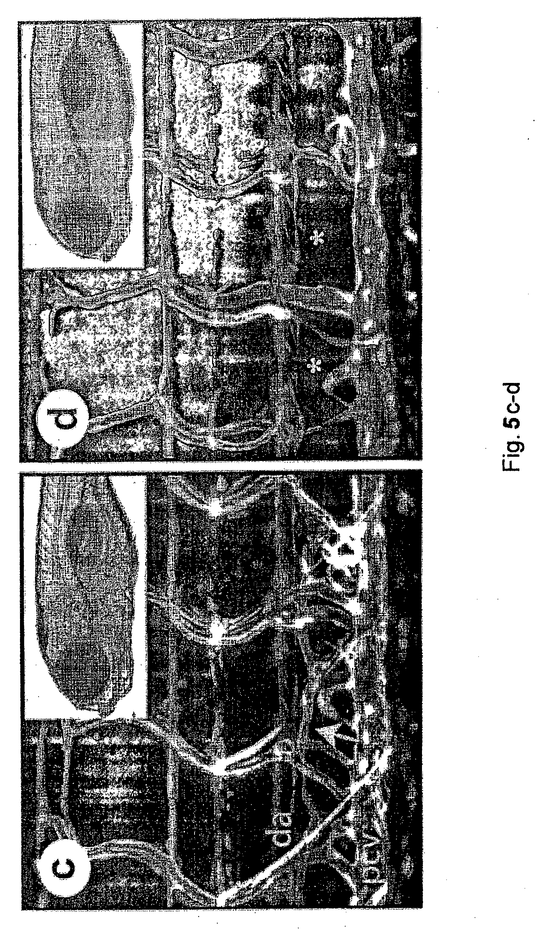

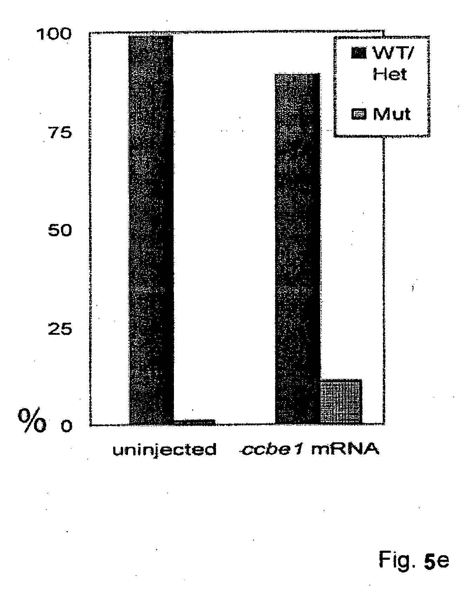

**Figure 5**: Sequence, phenocopy and rescue of *fof^{hu3613}.*
   **a** and **b**. Sequencing of *ccbe1* identified a transversion (T to A) corresponding to position 162 of the predicted protein, replacing aspartic acid (D) with glutamic acid (E) (affected codon underlined).
   **c** and **d**. Injection of a *ccbe1* ATG morpholinos phenocopies *fof* (**d**) (n=134/136 (splice MO n=20/59)); wildtype control in (c). Arrow=thoracic duct,asterix=absence of thoracic duct.
   **e**. *ccbe1* mRNA (400pg/embryo) rescued the *fof* phenotype. Embryos were selected for the presence of the thoracic duct, and subsequently genotyped (n=90 uninjected, 176 injected)

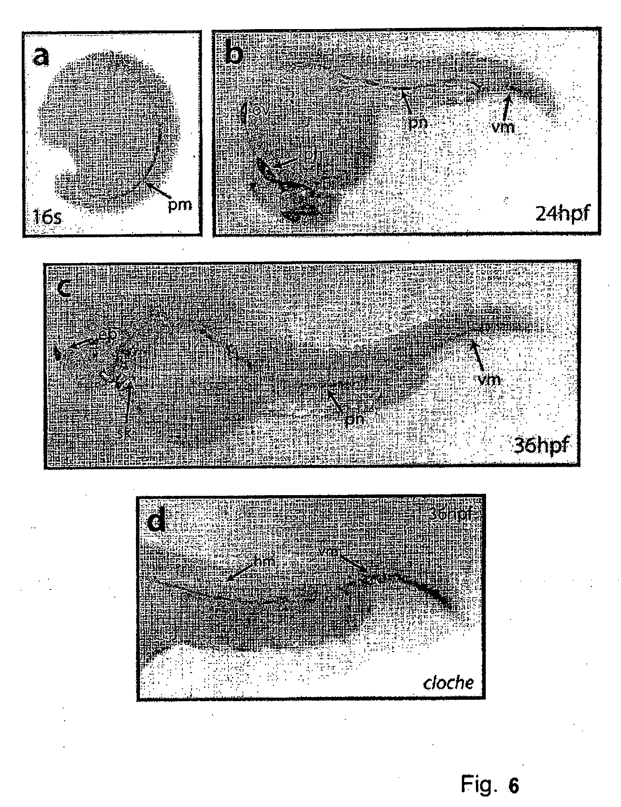

**Figure 6**: Embryonic expression of *ccbe1.*
   **a-c**. *ccbe1* is expressed in pronephric mesoderm (pm), pronephros (pn), ventral mesenchyme (vm), skin (sk), epiphysis (ep), and otic vesicle (ov) during development.
   **d**. Expression of was unaltered in *cloche* mutant embryos directly selected for the absence of trunk endothelial cells (dorsal to the yolk extension) (n=9).

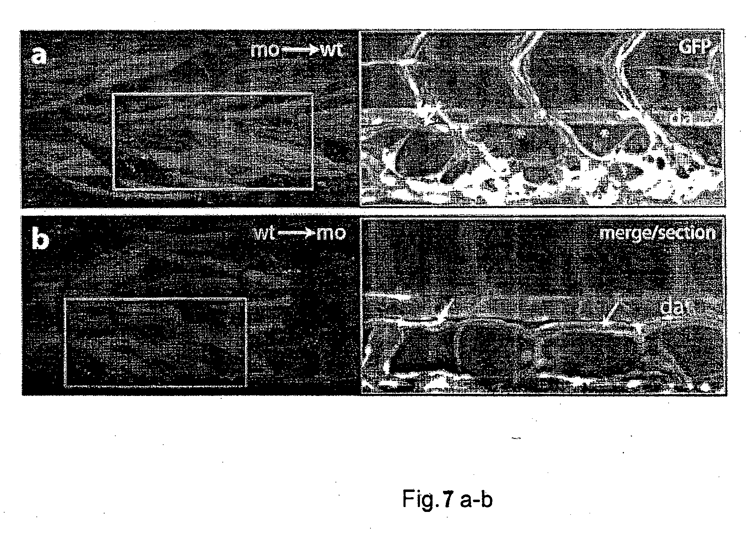

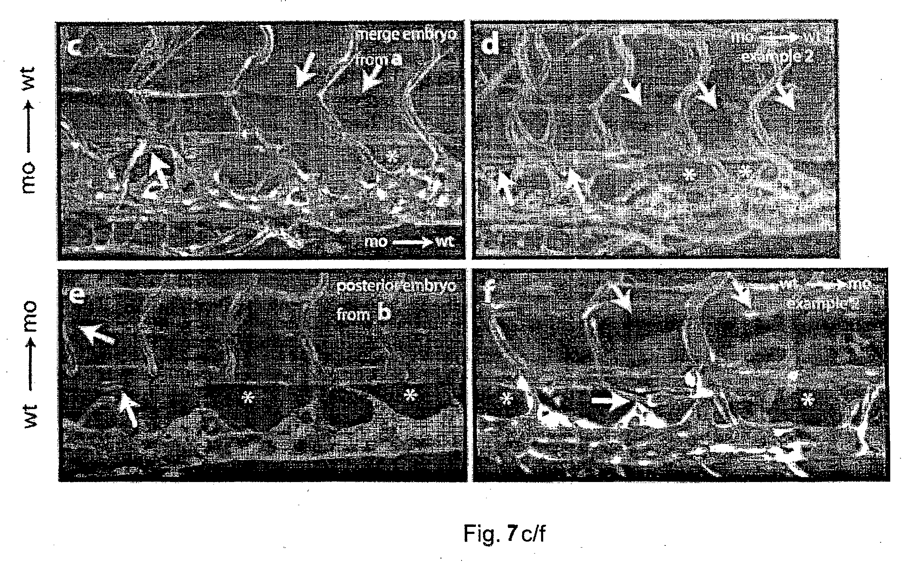

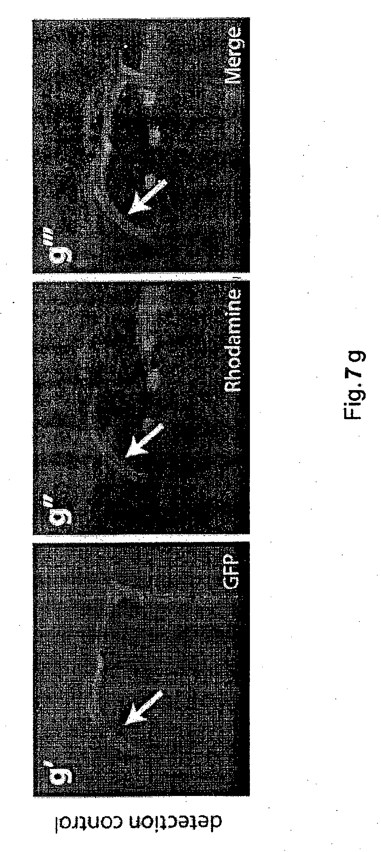

**Figure 7**: Transplantation assays.
   **a**. Morpholino injected cells (rhodamine labeled, left) transplanted into wildtype embryos disrupt thoracic duct formation (GFP, right, boxed area) when contributing to somitic mesoderm. In a total of 92 transplants, n=7/7 disrupted thoracic ducts were immediately ventral to labeled muscle cells (5dpf).
   **b**. Wildtype donor cells (rhodamine labeled, left) rescued thoracic duct formation by morphant cells (GFP/rhodamine in single confocal section, right, boxed area) when contributing to somatic mesoderm. n=134 embryos transplanted, n=7/7 with lymphatic fragments immediately ventral to grafted muscle, MO efficiency 98.5% (n=136; 5dpf).
      Arrows = presence, asterix = absence of thoracic duct fragments.
   c**.** Merged image of embryo from (**a**) indicating that large grafts of morphant muscle cells were required for disruption of thoracic duct.
   **d**. Second example of morphant cells grafted into wildtype embryos disrupting thoracic duct formation. Morphant muscle cells in the region of the horizontal myoseptum (yellow arrows) were associated with the absence of thoracic duct in this region (white asterix).
   **e**. The posterior of wt to morphant grafted embryo from (**b**) indicating the absence of thoracic duct in the absence of grafted muscle cells.
   **f**. Second example of wildtype cells grafted into morphant embryos rescuing thoracic duct formation by morphant cells. Wildtype muscle cells in the region of the horizontal myoseptum (yellow arrows) were associated with the presence of a small thoracic duct fragment (white arrow).
      Yellow arrows indicate the presence of grafted somitic mesoderm derived muscle, white arrows indicate the presence of thoracic duct cells, white asterix indicate the absence of thoracic duct cells in **c-f.**
   g. Transplanted endothelial cells were readily detectable by the presence of rhodamine label (red). (g'=GFP, g"=Rhodamine, g"'=Merge, Arrows indicate double labelled transplanted cell, single confocal section).

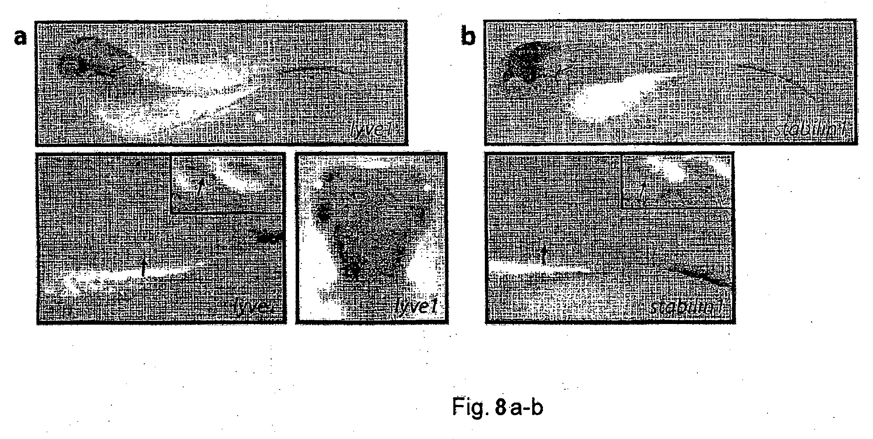

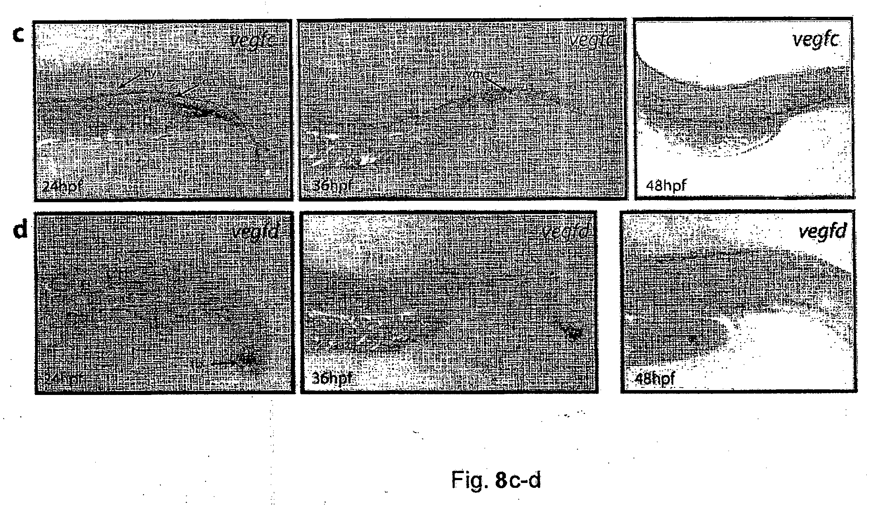

**Figure 8****:** Analysis of *lyve-, 1 stablilin-1, vegfc* and *vegfd* expression.
   *a. lyve-1* expression at 4dpf is readily observed in the pharyngeal region (arrow, upper) and caudal vein region, as well as in the dorsal aspect of the posterior cardinal vein (arrow, lower left), thoracic duct (with 2 days staining) (arrow, inset, lower left) and in the head (lower right).
   b. *stabilin-1* expression at 4dpf is observed in the pharyngeal region (arrow, upper), caudal vein region, the dorsal aspect of the posterior cardinal vein (arrow, lower) and thoracic duct (with 2 days staining) (arrow, inset, lower left) but is not observable in the head due to diffuse brain expression.
   c. *vegfc* expression at 24hpf, 36hpf and 48hpf is observed in the hypochord (hy), dorsal aorta (da) and ventral mesenchyme (vm) but expression levels are reduced a 48hpf.
   d. *vegfd* expression at 24hpf, 36hpf and 48hpf is observed in the tailbud (tb).

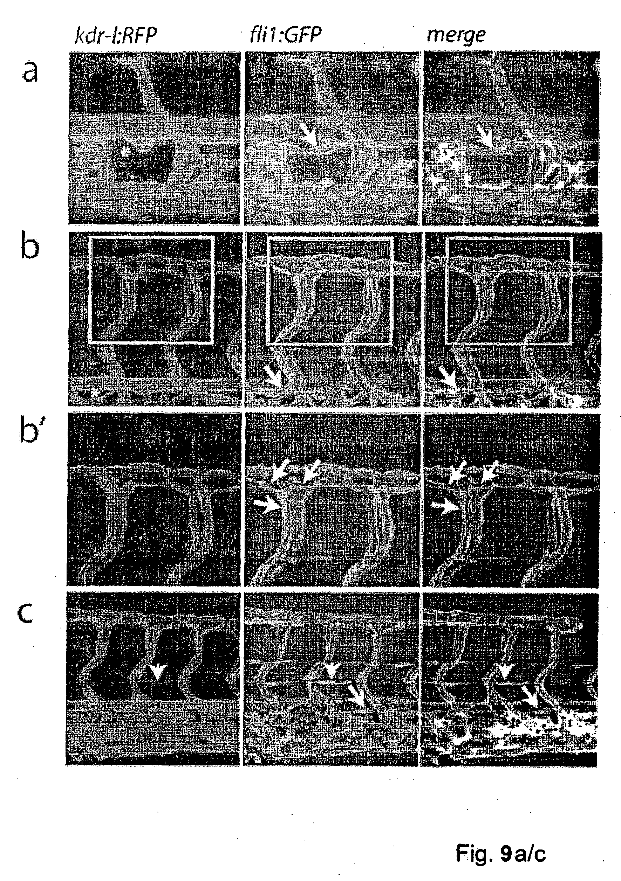

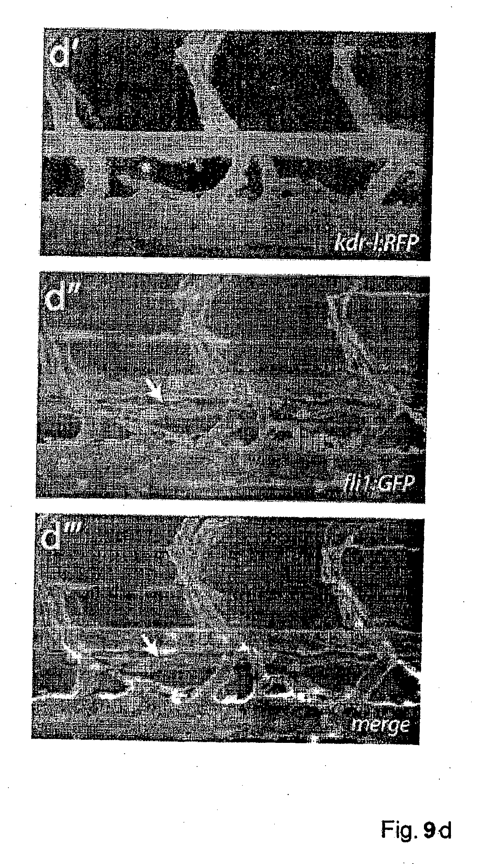

**Figure 9****:** The absence of *kdr-l (flk1)* expression in lymphatic vessels distinguishes the blood vasculature from lymphatic vasculature in *kdr-l:RFP, fli1:GFP* double transgenic animals.
   a. Expression of *kdr-l:RFP, fli1:GFP* and merge at 3.5dpf reveal that the earliest sprouting thoracic duct fragments are *kdr-l* negative and *fli1* positive.
   b. Intersegmental and dorsal longitudinal lymphatic vessels are *kdr-l* negative and *fli1* positive (arrow indicates the thoracic duct) (B'= boxed section from B, arrows indicate the DLLV and ISLV) (3.5dpf).
   c. Lymphatic vessels in the posterior are *kdr-l* negative and *fli1* positive (arrow) whilst cells in the parachordal region retain high *fli1* expression and weak residual *kdr-l* expression (arrowheads).
   d. RFP expression is absent from the thoracic duct (asterix, d') whilst GFP expression is present in the thoracic duct (arrow, d" and d'") at 6dpf.

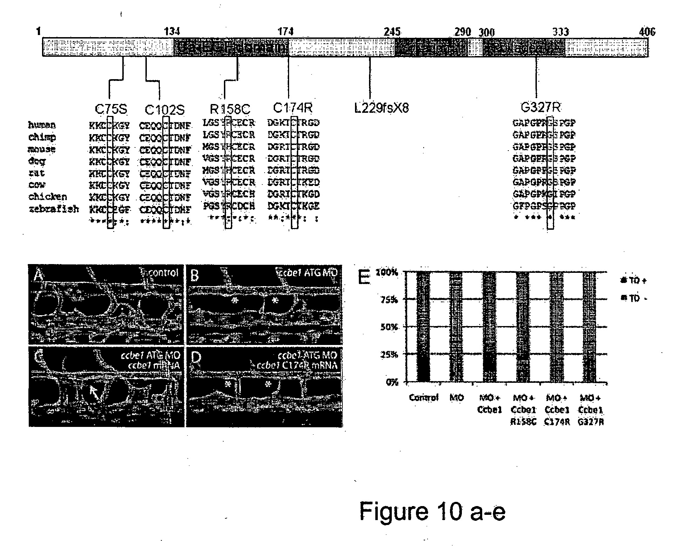

**Figure 10****:** Equivalent mutations in zebrafish Ccbe1 abolish normal gene function. Depicted on top is the human ccbe1 protein, the localization of the identified mutations, and homologous ccbe1 sequences in other organisms.
   **A** and **B.** Injection of *cebe1* ATG targeting morpholino (MO) (5ng/embryo) leads to a robust phenocopy of the *full of fluid* phenotype with absence of the thoracic duct in injected (**B**) compared with uninjected control (**A**) embryos at 5 dpf.
   **C** and **D.** The *ccbe1* ATG MO phenotype is rescued by injection of wildtype *ccbe1* mRNA (**C**) but not mRNA containing equivalent mutations to the human *CCBE1* mutations described here (example shown is failure of rescue with zebrafish *ccbe1* C166R mRNA (350pg/embryo) (**D**)).
   **E.** Summary of rescue experiments performed. Embryos were scored for the presence or absence of the thoracic duct (TD), data are shown for uninjected control (100% TD+, n=21 embryos scored), MO injected (0% TD+, n=45), MO + *ccbe1* (90% TD+, n=97), MO + *ccbe1* R150C mRNA (81% TD+, n=36), MO + *ccbe1* C166R mRNA (0% TD+, n=69) and MO + *ccbe1* G313R mRNA (0% TD+, n=73). These mutations are equivalent to human R158C, C174R and G327R respectively. The one letter amino acid annotation is used in this figure due to limited space.

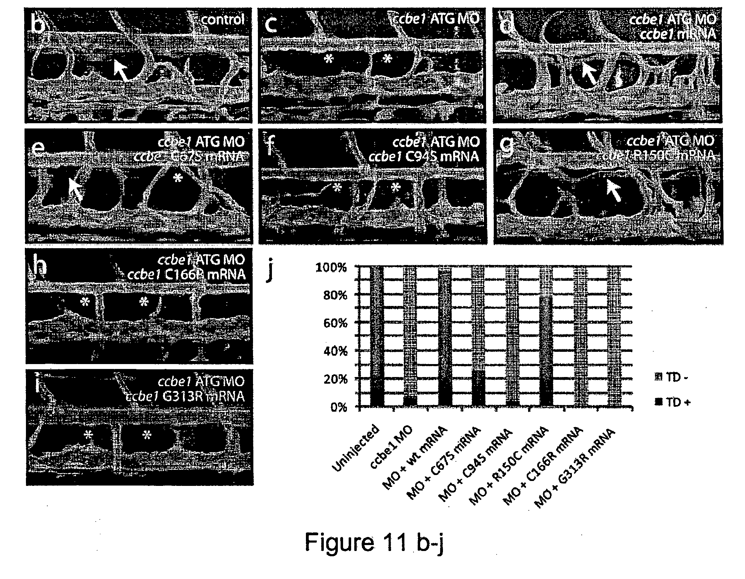

**Figure 11**: Mutations in CCBE1 abolish normal gene function. (a) Locations of mutations in conserved amino acids in CCBE1. Information on domain localization was obtained from UniProt (available on the world-wide web at uniprot.org).
   (b-j) Functional analysis of the mutations in the zebrafish ccbe1 model using the transgenic line TG(fli1a:gfp) y1, TG(kdr-l:ras-cherry)s916. Fli1a (green) is a marker for both blood and lymphatic endothelial cells, whereas Kdr-l (red) is expressed only in blood vessels12. (b,c) Injection of ccbe1 ATG-targeting morpholino (MO) (5 ng per embryo) led to a robust phenocopy of the ccbe1 mutant phenotype with absence (*) of the thoracic duct in injected (c) but not uninjected control (b) embryos 5 d postfertilization, (d-i) The ccbe1 ATG MO phenotype is robustly rescued by injection of wild-type ccbe1 mRNA (d) but not mRNA encoding the C67S (e), C94S (f), C166R (h) or G313R (i) substitutions. The mRNA encoding the R150C substitution was capable of rescue (g). Asterisk, lack of rescue; arrows, thoracic duct. mRNAs were all injected at 350 pg per embryo.
   (j) Summary of rescue experiments. Embryos were scored for the presence or absence of the thoracic duct (TD), and data are shown for uninjected control (100% TD+, n = 21 embryos scored), MO injected (6% TD+, n = 75), MO + ccbe1 mRNA (97% TD+, n = 117), MO + ccbe1 C67S mRNA (25% TD+, n = 108), MO + ccbe1 C94S mRNA (4% TD+, n = 96), MO + ccbe1 R150C mRNA (78% TD+, n = 57), MO + ccbe1 C166R RNA (0% TD+, n = 87) and MO + ccbe1 G313R RNA (0% TD+, n = 73). The mutations in e-i are equivalent to human C75S, C102S, R158C, C174R and G327R, respectively.

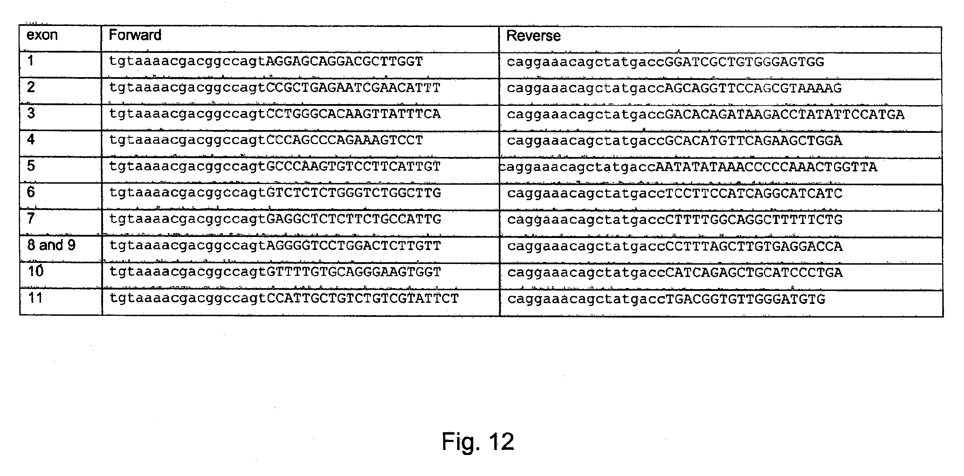

**Figure 12****:** Primers used for amplification of the coding exons of CCBE1. Primers are tagged with M13 sequences facilitating sequencing with universal M13 primers. The sequences written in capitals are the gene specific sequences.

### Examples

### Example 1:

Lymphatic vessels play important roles in fluid homeostatis, fat absorption, inflammation and cancer metastasis and develop in a dynamic process (called lymphangiogenesis) involving budding, migration and proliferation of lymphangioblasts. Using a genetic screen in zebrafish we identify *collagen and calcium-binding EGF domain-1 (ccbe1)* as indispensible for embryonic lymphangiogenesis. Ccbe1 acts at the same stage of development as Vegfc and is required for lymphangioblast budding and angiogenic sprouting from venous endothelium.

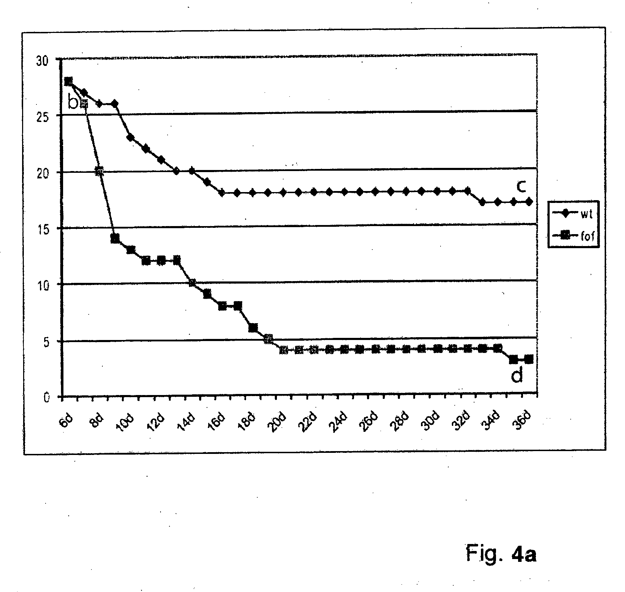

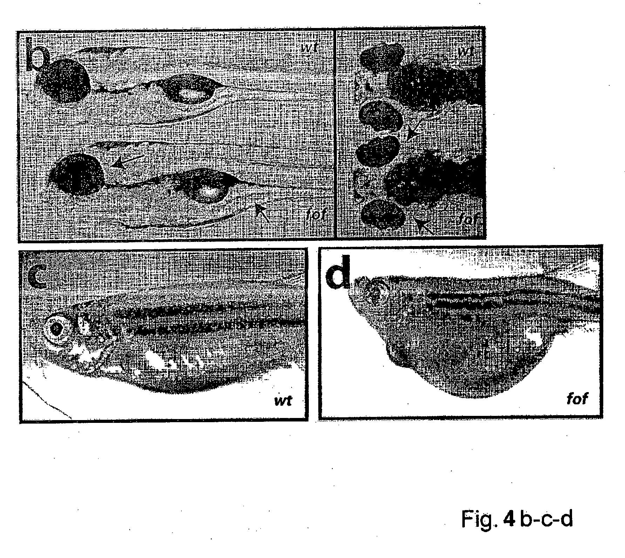

To identify novel regulators of lymphangiogenesis, we utilized the recently characterized zebrafish lymphatic vasculature^{1, 2} as a model. In a dedicated forward genetic screen, we isolated one mutant, *full of fluid* (*fof^{hu3613}*), which lacked the thoracic duct and the previously unidentified, intersegmental (ISLVs) and dorsal longitudinal lymphatic vessels (DLLVs) whilst retaining a patterned and functional blood vasculature (Fig. 1a-f). Mutants displayed edema, initiating in the lower intestine and around the eyes from 6 days post fertilization (dpf) and the few mutants that survived to 36dpf displayed severe edema (Fig. 1g and h, Fig. 4).

Genetic mapping of *fof* localized the mutation to chromosome 21 in an interval containing a single gene, *collagen and calcium binding EGF domain 1* (*ccbe1*) (Fig. 1i (upper)). *ccbe1* encodes a predicted secreted protein containing a signal peptide, a collagen domain, and a calcium binding EGF domain. Sequencing revealed a single coding mutation in the fourth exon of *ccbe1* changing an aspartic acid (D) to glutamic acid (E) in the calcium binding EGF domain (Fig. 5). The residue mutated (D162) is completely conserved in Ccbe1 proteins (Fig. 1i (lower left)). Significantly, an equivalent mutation (D1479E) in a calcium-binding EGF domain of Fibrilin1 is associated with loss-of-function phenotypes in humans³. Injection of *ccbe1* targeting morpholinos efficiently phenocopied *fof* at 5 dpf (Fig. 1i (lower right), Fig. 5) and the injection of *ccbe1* mRNA rescued lymphatic deficiency in *fof* mutants (Fig. 5), together demonstrating that *fof^{hu3613}* is a loss-of-function allele.

We observed restricted expression of *ccbe1* during development (Fig. 6) with expression in the pronephros and ventral mesenchyme at 24 hours post fertilization (hpf) (Fig. 2a). By 36 hpf, expression was detected in discrete zones along each somitic boundary, between the PCV and the horizontal myoseptum, as well as along the horizontal myoseptum itself (Fig. 2a). This expression was retained in *cloche^{hu2345}* mutant embryos (Fig. 6) and was therefore non-endothelial. At 48 hpf, *ccbe1* expression was restricted along the horizontal myoseptum (Fig. 2a). Intriguingly, this dynamic non-endothelial expression pattern correlates spatially and temporally with the migration routes of endothelial cells which bud from the PCV, migrate in close association with the somite boundaries and seed the horizontal myoseptum region from where lymphatic precursors later migrate^{1, 4}. Using embryonic transplantation assays we found that *ccbe1* function was indeed required in somitic mesoderm for adjacent thoracic duct formation and was sufficient for thoracic duct formation in otherwise *ccbe1*-deficient embryos when restricted to somitic mesoderm (Fig. 7). Taken together, the domain structure of Ccbe1 (signal peptide, collagen domain, calcium binding-EGF domain), non-endothelial expression and somitic mesodermal role indicate that *ccbe1* acts non-autonomously to promote embryonic lymphangiogenesis.

### Example 2:

To directly observe embryonic lymphangiogenesis, we produced a transgenic line by utilizing the promoter of *stabilin1*, a gene expressed in the same manner as *lyve1*⁷ in zebrafish (Fig. 8) and a marker of lymphatics in mammals ^{5, 6}. Expression of *stabilin1:YFP* was enriched in the PCV and venous-derived endothelial cells, including endothelial cells at the horizontal myoseptum by 48hpf. At later stages, expression was largely restricted to lymphatic and venous endothelium (Fig. 2b). Using live imaging, *stabilin1*-positive cells were observed to bud directly from the PCV to the horizontal myoseptum from approximately 36 hpf (Supplementary Movie 1). Between 48 and 72 hpf, these cells remained in this region before migrating dorsally, to contribute to the DLLV, or ventrally, to contribute to the thoracic duct (Supplementary Movie 2). In *ccbe1* morphants, *stabilin1*-expressing cells failed to bud from the PCV (Fig. 2b, Supplementary Movie 3).

Vegfc/Vegfr3 signaling is required for the initiation of lymphangioblast budding in mammals ^{8, 9}. Zebrafish *vegfc* but not *vegfd* expression is found immediately dorsal to the PCV prior to and concurrent with the budding of *stabilin1*-expressing cells (Fig. 8) ¹⁰. We injected *vegfc* targeting morpholinos² and found that *vegfc* was required for budding of *stabilin1*-positive cells at precisely the stage requiring *ccbe1* (fig. 2b, Supplementary Movie 4).

As observed for *stabilin1* expressing cells, previous studies have described budding of cells from the PCV to the horizontal myoseptum to form a structure dubbed the parachordal vessel⁴ from which lymphatic precursors later derive¹. We found that the *fli1*-expressing cells in the parachordal region do not form a vascular tube or support blood flow (Fig. 2c). Rather, by taking advantage of the absence of *kdr-like (flk1)*¹¹ expression in lymphatic vessels (Supplementary Fig. 6), we found that the majority of these cells migrate away from the horizontal myoseptum to contribute to the lymphatic vasculature and are present only transiently at the horizontal myoseptum during development (Fig. 2d, Supplementary Movie 5). Therefore, these cells do not constitute a blood vessel and function primarily as lymphatic vascular precursors (lymphangioblasts).

We next examined *fli1*-expressing lymphangioblasts in the absence of arteries and derivatives, in a scenario experimentally induced by suppressing *phospholipase Cgamma 1 (plcg)* function¹². In *plcg* morphants, *fli1* expressing lymphangioblasts budded from the PCV in the absence of intersegmental vessels, but budding was absent in *fof* mutant and *vegfc* morphant embryos (Fig. 2e). *tie2* expression identifies venous derived sprouts in wildtype embryos (48hpf). We found that these sprouts were absent in *fof* mutants and *vegfc* morphants, however, these sprouts were also absent in *plcg* morphants indicating that they are a cellular population distinct from lymphangioblasts (Fig. 2f). Hence, we developed a double transgenic line (*flt1:YFP, kdr-l:RFP*), which unmasks the venous or arterial origins of individual cells of the trunk vasculature. Venous-derived intersegmental vessel sprouts were absent in *ccbe1* and *vegfc* morphants (Fig. 2g). Hence, both angiogenic sprouting and lymphangiogenic budding from the PCV require *ccbe1* and *vegfc*, but only angiogenic sprouting requires *plcg*, genetically separating the two processes.

Taken together, these data demonstrate that, during embryogenesis, *ccbe1* and *vegfc* act at the level of both angiogenic sprouting and lymphangiogenic budding from venous endothelium. Alongside *uegfc ⁹* and *prox1 ^{13, 14}* knockout mice, the *full of fluid* mutant represents just the third vertebrate loss-of-function mutant which leads to the specific loss of the embryonic lymphatic vasculature.

### Example 3:

Disturbances of lymphangiogenesis usually cause disruption of the drainage of interstitial fluids into the cardiovascular system, resulting in lymphedema, chylothorax or pleural effusion, chylous ascites, and angiectasias of lymph vessels in intestines and other organs. Signs of lymph-vessel dysplasias are commonly limited to the limbs. In 1989, an inbred family was described in which four mentally retarded members had a widespread congenital lymphatic malformation syndrome with limb lymphedema, and lymphangiectasias of the intestine and at a later age also of the lungs (Hennekam, R.C. et al. Am. J. Med. Genet. 34, 593-600 (1989). In addition, affected individuals had unusual facial characteristics (flat face, flat and broad nasal bridge, hypertelorism) thought to reflect the extent of early intrauterine facial lymphedema. Subsequently, subjects with lymphangiectasias in pleura, pericardium, thyroid gland and kidney and with hydrops fetalis were described (Van Balkom, I.D. et al. Am. J. Med. Genet. 112, 412-421 (2002) and Bellini, C. et al. Am. J. Med. Genet. 120A, 92-96 (2003)). The entity was designated lymphedema-lymphangiectasia-mental retardation or Hennekam syndrome (MIM 235510). Occurrence of affected siblings, equal occurrence among sexes and frequent consanguinity indicated autosomal recessive inheritance.

We collected blood samples from a series of 27 subjects with Hennekam syndrome born to 22 families. None of the subjects carried mutations in FLT4, FOXC2 or SOX18. Mutation analysis of the human *Ccbe1* gene was performed by PCR amplification of all 11 coding exons of Ccbe1 and subsequent sequencing using the BIGdye terminator kit and ABI3700 sequencer (Applied Biosystems). Primers for amplifying the coding exons are shown in Figure 12. This revealed homozygous mutations in 5 Hennekam Syndrome (HS) patients of which three patients (JP, AK and KB) originate from a small isolated village in the Netherlands known for a high prevalence of inbreeding. Pedigree analysis had shown the parents of one of them to be consanguineous and all three patients to be related. Two further patients (MM and TS) from highly consanguineous parents were further included in the analysis.

Both the mutations in JP, AK and KB (p.Cys75Ser) and MM (p.Cys102Ser) are N-terminal of the putative calcium binding EGF domain. However, this region also displays some EGF-like sequences and disruption of conserved cysteines may affect secondary and/or tertiary structures. The mutation p.Gly258Arg in TS is predicted to disrupt the glycine backbone in the putative collagen helix of CCBE1.

Two additional patients (GV, LH) born to non- consanguineous parents were subsequently identified with mutations in the Ccbe1 gene. LH carried a maternally inherited insertion of 1 nucleotide, c.683_684insT, which results in a frameshift mutation. In addition, GV carried a paternally inherited missense mutation p.Arg158Cys. LH was heterozygous for c.683_684insT and for p.Cys174Arg. Both missense mutations p.Arg158Cys and p.Cys174Arg are in the calcium binding EGF domain, the first introducing another cysteine that might interfere with proper folding of the protein in the domain, and the latter disrupting a cysteine residue predicted to form disulfide bonds and required for the secondary structure of this domain (figure 3). All mutations detected in the patients were absent in a control population of healthy individuals of Western European (n=100) or Arabic (UAE) (n=97) descent. None of the other patients from 17 families in the total HS group harboured a CCBE1 mutation.

The function of Ccbe1 was studied in the zebrafish model *full of fluid (fof).* This model is homozygous for a *ccbe1* mutation and lacks the thoracic duct as well as intersegmental and dorsal longitudinal lymphatic vessels. Mutants develop lymphedema, but retain a largely normal cardiovascular system. This model is suitable to test the pathogenicity of the missense mutations identified in the human HS patients.

All zebrafish strains were maintained in the Hubrecht Institute using standard husbandry conditions. Animal experiments were approved by the Animal Experimentation Committee of the Royal Netherlands Academy of Arts and Sciences (DEC). The transgenic line used was the *TG(fli1a:gfp)*^{*y*1,} *TG(kdr-l:ras-cherry)^{s916}* double transgenic line (Hogan et al. (2009). Nat. Genet. 41, 396-398). Homologous mutations were introduced in zebrafish *ccbe1*-Myc pCS2+ (p.Cys166Arg and p.Gly313Arg) or *ccbe1*-pCS2+(p.Arg150Cys). Each of the mRNAs tested was synthesised simultaneously with wildtype control mRNAs (synthesis as previously described in (Hogan et al. (2009). Nat. Genet. 41, 396-398) and injected (350pg/embryo) separately into embryos already injected with a *ccbe1* ATG targeting morpholino at the one cell stage at a concentration of 5ng/embryo. Injection of wildtype *ccbe1* mRNA did reliably rescue the *fof* phenotype, as evidenced by the presence of thoracic duct fragments just ventral to the dorsal aorta, or by a complete rescue of thoracic duct formation.

Two of the three mutant mRNAs tested (p.Cys166Arg and p.Gly313Arg, which are equivalent to human p.Cys174Arg and p.Gly327Arg, respectively) were not able to confer any rescue (Figure 10B-D and Figure 11). Unexpectedly, mutant p.Arg150Cys (equivalent to human p.Arg158Cys) did rescue the *fof* phenotype. This indicates that at least some protein function is preserved, but does not exclude pathogenicity. The p.Arg150Cys mutation may result in drastically reduced activity of the protein, but when administered in excess by high dose mRNA injection in zebrafish may still deliver sufficient function to rescue the *fof* phenotype.

The mutations in CCBE1, mostly missense mutations, act as recessive mutations and heterozygous carriers are without any noticeable sign or symptom. Apparently the mutations result in proteins that are non-functional or hypomorph and do not have a dominant-negative effect. In zebrafish, the ccbe1 mutation is predominantly lethal; only a small number of mutated zebrafish survive (Hogan et al. (2009). Nat. Genet. 41, 396-398). In humans there is no increased miscarriage rate or chance for still birth and neonatal death in families with HS, and almost all affected patients survive into adulthood (Van Balkom et al (2002). Am. J. Med. Genet. 112, 412-421). One may argue that this difference in survival rate might be due to the difference in mutations: mutations in humans may leave a little functionality while the zebrafish mutation completely abolishes protein function. If indeed p.Arg158Cys is pathogenic due to reduced activity, this would favour this theory. However, p.Cys174Arg showed a complete inability to rescue the fof phenotype. In addition, the fof mutation itself, p.Asp162Glu, is also located within the EGF binding domain and not clearly different from some of the human mutations reported here. Therefore, alternative explanations for the difference in lethality may be differences in lymph vessel anatomy between zebrafish and humans, differences or redundancy in the function of CCBE1 or other genes involved in lymphangiogenesis in humans.

### Material and Methods

Zebrafish strains including previously described transgenic lines ^{21, 26} were maintained using standard husbandry conditions. Mutagenesis was performed as previously described ²⁷, F2 progeny were incrossed and F3 screened for the presence of the thoracic duct. Genetic mapping was performed as previously described ²⁸, primer sequences and genotyping approaches can be found in full methods online. *ccbe1* was targeted with start codon and splice site mopholinos (Genetools, LLC) at 2.5 or 5ng/embryo, *plcg* morpholino (Open biosystems) was injected at 5ng/embryo. Morpholino sequences and construct details can be found in the supplementary methods. Imaging and transplantation assays were performed as previously described ²⁸ and are described in the supplementary methods. A citrine-neomycin cassette was recombined into BAC (bacterial artificial chromosome) clones using Red/ET Recombination Technology (Gene Bridges) and an IS*ce*I meganuclease site integrated as previously described ²⁹. *In situ* hybridisation was performed as previously described ²⁸ with probes synthesized as described in the supplementary methods.

### Zebrafish strains and screen

All zebrafish strains were maintained in the Hubrecht Institute using standard husbandry conditions. The zebrafish is originally geographically distributed in South-East Asia. The strains were all cultured laboratory strains. Animal experiments were approved by the Animal Experimentation Committee of the Royal Netherlands Academy of Arts and Sciences (DEC). Published transgenic lines used were *TG(fli1a:gfp)^{y1}* (Lawson, N. D. & Weinstein, B. M. In vivo imaging of embryonic vascular development using transgenic zebrafish. Dev Biol 248, 307-18 (2002)) and *TG(kdr-l:ras-cherry)^{s896}* (Chi, N. C. et al. Foxn4 directly regulates tbx2b expression and atrioventricular canal formation. Genes Dev 22, 734-9 (2008)), *(*k*dr-l:RFP*) (also known as *flk1:ras-cherry ^{s896}*) (Bussmann, J., Bakkers, J. & Schulte-Merker, S. Early endocardial morphogenesis requires Scl/Tal1. PLoS Genet 3, e140 (2007)). The *cloche^{hu2345}* allele has been previously described (Herpers, R., van de Kamp, E., Duckers, H. J. & Schulte-Merker, S. Redundant roles for sox7 and sox18 arteriovenous specification in zebrafish. Circ Res 102, 12-5 (2008)).

ENU mutagenesis was performed as previously described for the creation of the Hubrecht Institute target selected mutagenesis library (Wienholds, E., Schulte-Merker, S., Walderich, B. & Plasterk, R. H. Target-selected inactivation of the zebrafish rag1 gene. Science 297, 99-102 (2002)). F1 progeny of mutagenised males were outcrossed to the *fli1:GFP* strain to create approximately 300 F2 families, which were then incrossed. F3 progeny were screened for the presence of the thoracic duct. Genetic mapping and genotyping

Bioinformatic construction of the genomic region was performed using the Ensembl database (available on the world wide web at ensembl.org), release 44, April 2007. Meiotic mapping of the *full of fluid* mutation was performed using standard simple sequence length polymorphisms. The primers used for SSLP and SNP markers depicted in Fig. 2 were as follows: z62970; 5'-ACACTGTAAACCAGACGGCC-3' and 5'-TCAGTCAAGCGCACGTAATC-3', SNP1; 5'-GCCTCCACACCTGGATAAC-3' and 5'-CCTGGGATATTAGCTTGCAG-3', CA1; 5'-ACTGACCCCAAACTTTTGAC-3' and 5'-CATGTCTACAGCCTGAATGC-3' , CA2; 5'-ATTTGCGTCAATGCTAACAC-3' and 5'-CAGTCTGCTCAGGTTGGAG-3'.

Total RNA (1µg) from wildtype and mutant embryos was reverse transcribed using 12.5µM random hexamers, 8mM MgCl₂, 1mM each dNTP, 1U/µl RNase inhibitor, and 10U/µl MMuLV reverse transcriptase (Promega). PCR followed by sequencing of wildtype and mutant cDNA was performed with the primers: 5'-GCGCTGAACTTCAAGACTG-3' and 5'-ATCATCCTCCAGGTAGAAGC-3', 5'-AGAAACCATATTGCCTGGAC-3' and 5'-TTTGATATGCGACAGGTCAG-3', 5'-GGCTCTCCTGGACAGATG-3' and 5'-ATTCAGCCTTCTTTCCTCAG-3'. Subsequent genotyping of *ccbe1* mutants was performed on individual embryos by utilizing an informative *ccbe1* intronic CA repeat marker with the primers: 5'-CAACTTTCTGTCCCTCACAC-3' and 5'-GCGTGTCCTCATTTACTTTG-3'

### Morpholino oligos and mRNA synthesis

The *ccbe1* start codon targeting MO (ATG MO 5'-CGGGTAGATCATTTCAGACACTCTG-3') (Genetools, LLC) was injected at a concentration of 2.5 or 5ng/embryo. The *ccbe1* splice site targeting MO (5'-ACAGCACAGCACTTTACCTGTCTAC-3') (Genetools, LLC) was injected at a concentration of 5ng/embryo. The *plcg* MO (5'-ATTAGCATAGGGAACTTACTTTCG-3') (Open biosystems) was injected at a concentration of 5ng/embryo. The full length *ccbe1* coding sequence was PCR amplified from the template EST clone EE696184 using the following primer pair: 5'-gcgcgaattcaccATGATCTACCCGGGCAGAGG-3' and 5'-gcgcctcgagTCAAACCGGCCAATCGGGAT-3' and cloned into pCS2+ (Turner, D. L. & Weintraub, H. Expression of achaete-scute homolog 3 in Xenopus embryos converts ectodermal cells to a neural fate. Genes Dev 8, 1434-47 (1994)) using the *Eco*RI and *Xho*I restriction sites.

### Generation of transgenic lines

A citrine-neomycin cassette was recombined using Red/ET Recombination Technology (Gene Bridges) into the BAC (bacterial artificial chromosome) clones DKEY-182E1 (*stabilin1*) or DKEY-256I8 (*flt1*) using the homology arm tagged PCR primers: *stabilin1* forward primer;
5'-tgtttgttgttacatgtttatctgactaattctctggctttgagggagtaACCATGGTGAGCAAGGGC GAGGAG-3',
stabilin1 reverse primer;
5'-gaatctgtgcagtccacagtactgtaagtcccaaaacgagaagataagaTCAGAAGAACTCGTCA AGAAGGCG-3',
fltl forward primer; 5'-tccataggtattccttcatctccaaacaaacaccctcaagcaagaccaagACCATGGTGAGCAAGG GCGAGGAG-3', flt1 reverse primer;
5'-gtcagaacacgtcctgacagtccaaatatcatcacaaataatatatcgaaTCAGAAGAACTCGTCA AGAAGGCG-3'.

In addition, an ISceI meganuclease site was integrated into the clone backbone for ease of transgenesis as previously described (Kimura, Y., Okamura, Y. & Higashijima, S. alx, a zebrafish homolog of Chx10, marks ipsilateral descending excitatory interneurons that participate in the regulation of spiral locomotor circuits. J Neurosci 26, 5684-97 (2006)).

### In situ hybridization

*In situ* hybridisation was performed as previously described (Bussmann, J., Bakkers, J. & Schulte-Merker, S. Early endocardial morphogenesis requires Scl/Tal1. PLoS Genet 3, e140 (2007)) .The *tie2* probe has been previously described (Lyons, M. S., Bell, B., Stainier, D. & Peters, K. G. Isolation of the zebrafish homologues for the tie-1 and tie-2 endothelium-specific receptor tyrosine kinases. Dev Dyn 212, 133-40 (1998)). *ccbe1* probe was synthesized by *in vitro* transcription from the *EcoR*I digested full length cDNA in pCS2+ using the T7 RNA polymerase (Promega). *vegfc* and *vegfd* riboprobes were made by first cloning their coding sequences into the pCS2+ vector using PCR from template clone BC114253 (*vegfc*) (Open Biosystems) or cDNA with the primers: *vegfc*; 5'-gcgcgaattcaccATGCACTTATTTGGATTTTC-3' and 5'-gcgcctcgagTTAGTCCAGTCTTCCCCAGT-3', *vegfd*; 5'-gcgcgaattcaccATGAAGAAACAGAAATGTGC-3', 5'-gcgcctcgagTCACGTATAGTGTAGTCTGT-3', followed by cloning into the *EcoR*I and XhoI restriction sites and *in vitro* transcription from EcoRI digested template using the T7 RNA polymerase (Promega). *lyve-1* probe clone (corresponding to ENSDARG00000062483) was a gift from Exelixis and RNA was synthesized by digestion with EcoRV and transcription with SP6 polymerase. *stabilin-1* probe was synthesized by first PCR amplifying an exonic fragment with the PCR primers: 5'- CACTGATGTAGTGCTGGTTG-3' and 5'-GGATCCATTAACCCTCACTAAAGGGAACACAGAAGGGCTGTCAAAC-3' and then synthesizing RNA using T3 RNA polymerase (Promega).

### Imaging

Embryos were mounted in 0.5-1% low melting point agarose in a culture dish with a cover slip replacing the bottom. Imaging was performed with a Leica SP2 or SP5 confocal microscope (Leica Microsystems, available on the world wide web at leica-microsystems.com/) using a 20x or 40x objective with digital zoom. Timelapse analysis was compiled using ImageJ software (available on the world wide web at rsb.info.nih.gov/ij/). Time points were recorded every 20 minutes for the stated time period. A heated stage maintained the embryos at approximately 28.5 °C.

### Transplantation

Transplantation was performed essentially as previously described (Ho, R. K. & Kane, D. A. Cell-autonomous action of zebrafish spt-1 mutation in specific mesodermal precursors. Nature 348, 728-30 (1990)). Briefly, wildtype donor embryos of the genotype *TG(fli1:GFP)* were injected with 70KDa Tetramethyl Rhodamine (TAMRA) (Molecular Probes) with or without *ccbe1* ATG MO (5ng/embryos) at the one cell stage and utilized as donors at pre-dome stages. More than 10 cells were transferred from donor to recipient embryos between sphere and 30% epiboly stages. Embryos were first scored for the presence or absence of lymphatic fragments in the thoracic duct region and then analysed for relative grafted cell position. Only morphologically normal embryos were examined.

### Literature cited in the Examples

1. Yaniv, K. et al. Live imaging of lymphatic development in the zebrafish. Nat Med 12, 711-6 (2006).
2. Kuchler, A. M. et al. Development of the zebrafish lymphatic system requires VEGFC signaling. Curr Biol 16, 1244-8 (2006).
3. FBN1-mutations-database. (available on the world wide web at umd.be:2030/W_FBN1).
4. Isogai, S., Lawson, N. D., Torrealday, S., Horiguchi, M. & Weinstein, B. M. Angiogenic network formation in the developing vertebrate trunk. Development 130, 5281-90 (2003).
5. Prevo, R., Banerji, S., Ni, J. & Jackson, D. G. Rapid plasma membrane-endosomal trafficking of the lymph node sinus and high endothelial venule scavenger receptor/homing receptor stabilin-1 (FEEL-1/CLEVER-1). J Biol Chem 279, 52580-92 (2004).
6. Salmi, M., Koskinen, K., Henttinen, T., Elima, K. & Jalkanen, S. CLEVER-1 mediates lymphocyte transmigration through vascular and lymphatic endothelium. Blood 104, 3849-57 (2004).
7. Banerji, S. et al. LYVE-1, a new homologue of the CD44 glycoprotein, is a lymph-specific receptor for hyaluronan. J Cell Biol 144, 789-801 (1999).
8. Makinen, T. et al. Inhibition of lymphangiogenesis with resulting lymphedema in transgenic mice expressing soluble VEGF receptor-3. Nat Med 7, 199-205 (2001).
9. Karkkainen, M. J. et al. Vascular endothelial growth factor C is required for sprouting of the first lymphatic vessels from embryonic veins. Nat Immunol 5, 74-80 (2004).
10. Covassin, L. D., Villefranc, J. A., Kacergis, M. C., Weinstein, B. M. & Lawson, N. D. Distinct genetic interactions between multiple Vegf receptors are required for development of different blood vessel types in zebrafish. Proc Natl Acad Sci U S A 103, 6554-9 (2006).
11. Bussmann, J., Lawson, N., Zon, L. & Schulte-Merker, S. Zebrafish VEGF receptors: a guideline to nomenclature. PLoS Genet 4, e1000064 (2008).
12. Lawson, N. D., Mugford, J. W., Diamond, B. A. & Weinstein, B. M. phospholipase C gamma-1 is required downstream of vascular endothelial growth factor during arterial development. Genes Dev 17, 1346-51 (2003).
13. Wigle, J. T. et al. An essential role for Prox1 in the induction of the lymphatic endothelial cell phenotype. Embo J 21, 1505-13 (2002).
14. Wigle, J. T. & Oliver, G. Prox1 function is required for the development of the murine lymphatic system. Cell 98, 769-78 (1999).

## Claims

1. A method for testing whether a compound is capable of inhibiting the development of lymphatic channels or lymphangiogenesis and/or the migration of lymphangioblasts in an non-human animal, a non-human embryo or a cell culture, comprising the steps of:
a. administering a compound capable of interacting with a Ccbe1 gene, a transcript thereof, or a ccbe1 protein to a non-human animal, a non-human embryo or a cell culture;
b. determining whether said compound inhibits the development of lymphatic channels, lymphangiogenesis and/or migration of lymphangioblasts in said non-human animal, a non-human embryo or a cell culture.

2. A method according to claim 1, wherein said compound is selected from the group consisting of an antibody directed against Ccbe1 protein or functional fragment thereof, and an antisense agent against Ccbe1.

3. A method for testing whether a compound is capable of inhibiting the development of lymphatic channels or lymphangiogenesis and/or the migration of lymphangioblasts in an non-human animal, a non-human embryo, or a cell culture, comprising the steps of:
a. administering a compound to a non-human animal, a non-human embryo, or a cell culture, wherein said non-human animal, said non-human embryo, or cell culture do not express the Ccbe1 protein in a functional level;
b. inducing the expression of Ccbe1 protein in cells of said non-human animal or said non-human embryo; and
c. determining whether said compound inhibits the development of lymphatic channels, lymphangiogenesis and/or migration of lymphangioblasts in said non-human animal, said non-human embryo or said cell culture.

4. A method according to any one of claims 1-3, wherein said animal or said embryo is transparent.

5. A method according to any one of claims 1-4, wherein said animal or said embryo is genetically modified, wherein the modification comprises the transgenic expression of stabilin1, tie2, lyve1 and/or fli1 fused to a GFP or a derivative thereof.

6. A method according to any one of claims 3-5, wherein said animal or said embryo is genetically modified such that the ccbe1 gene is knocked out.

7. A method according to any one of claims 1-6, wherein said animal or said embryo is a fish.

8. A Ccbe1 antisense agent comprising the base sequence of:
5'-CGGGTAGATCATTTCAGACACTCTG-3',
5'-ACAGCACAGCACTTTACCTGTCTAC-3' or
5'-ATTAGCATAGGGAACTTACTTTCG-3' for use in a method for treating cancer.

9. An antisense agent having between 15-40 nucleobases in length comprising at least 8 consecutive nucleobases complementary and is capable of hybridizing to a nucleotide sequence encoding ccbe1, preferably of the base sequence as depicted in SEQ ID NO:35 for use in a method for treating cancer.

10. A method of determining whether an individual is a carrier of, or is suffering from, or at risk of suffering from, a congenital lymph vessel disorder, comprising providing a sample from said individual; determining the presence of an alteration in the sequence of the Ccbe1 gene, and determining that the individual is a carrier, or is suffering from, or at risk of suffering from, the congenital lymph vessel disorder if said alteration is present.

11. The method according to claim 10, wherein said lymph vessel disorder is selected from Meige, Nonne-Milroy and Hennekam syndrome.

12. The method according to claim 10 or 11, wherein the alteration of the Ccbe1 gene results in amino acid changes C75S, C102S, C174R, L229fsX8 and/or G327R.

13. A composition comprising Ccbe1 protein or a functional fragment thereof capable of inducing lymph vessel formation, or comprising a nucleic acid encoding Ccbe1 or a functional fragment thereof capable of inducing lymph vessel formation, for use as a medicament for the treatment of a lymph vessel disorder.

14. The composition for use according to claim 13, wherein said lymph vessel disorder is a congenital lymph vessel disorder or an acquired lymph vessel disorder selected from lymphedema, cancer, and a disorder resulting from the removal or irradiation of lymphatic vessels.

## Patentansprüche

1. Verfahren zum Testen, ob eine Verbindung in der Lage ist, die Entwicklung lymphatischer Kanäle oder Lymphangiogenese und/oder die Migration von Lymphangioblasten in einem nicht-menschlichen Tier, einem nicht-menschlichen Embryo oder einer Zellkultur zu inhibieren, umfassend die Schritte:
a. Verabreichen einer Verbindung, die in der Lage ist, mit einem Ccbe1-Gen, einem Transkript davon oder einem Ccbe1-Protein zu interagieren, an ein nicht-menschliches Tier, einen nicht-menschlichen Embryo oder eine Zellkultur;
b. Bestimmen, ob die Verbindung die Entwicklung lymphatischer Kanäle, Lymphangiogenese und/oder Migration von Lymphangioblasten in dem nicht-menschlichen Tier, einem nicht-menschlichen Embryo oder einer Zellkultur inhibiert.

2. Verfahren nach Anspruch 1, wobei die Verbindung ausgewählt ist aus der Gruppe, bestehend aus einem Antikörper, gerichtet gegen das Ccbe1-Protein oder ein funktionelles Fragment davon, und einem Antisense-Agens gegen Ccbe1.

3. Verfahren zum Testen, ob eine Verbindung in der Lage ist, die Entwicklung lymphatischer Kanäle oder Lymphangiogenese und/oder die Migration von Lymphangioblasten in einem nicht-menschlichen Tier, einem nicht-menschlichen Embryo oder einer Zellkultur zu inhibieren, umfassend die Schritte:
a. Verabreichen einer Verbindung an ein nicht-menschliches Tier, einen nicht-menschlichen Embryo oder eine Zellkultur, wobei das nicht-menschliche Tier, der nicht-menschliche Embryo oder die Zellkultur das Ccbe1-Protein nicht in einer funktionellen Ebene exprimieren;
b. Indizieren der Expression von Ccbe1-Protein in Zellen des nicht-menschlichen Tieres oder des nicht-menschlichen Embryos; und
c. Bestimmen, ob die Verbindung die Entwicklung lymphatischer Kanäle, Lymphangiogenese und/oder Migration von Lymphangioblasten in dem nicht-menschlichen Tier, dem nicht-menschlichen Embryo oder der Zellkultur inhibiert.

4. Verfahren nach einem der Ansprüche 1-3, wobei das Tier oder der Embryo transparent ist.

5. Verfahren nach einem der Ansprüche 1-4, wobei das Tier oder der Embryo genetisch modifiziert ist, wobei die Modifizierung die transgene Expression von stabilin1, tie2, lyvel und/oder fli1, fusioniert mit einem GFP oder einem Derivat davon, umfasst.

6. Verfahren nach einem der Ansprüche 3-5, wobei das Tier oder der Embryo genetisch modifiziert ist, so dass das Ccbe1-Gen außer Gefecht gesetzt wird.

7. Verfahren nach einem der Ansprüche 1-6, wobei das Tier oder der Embryo ein Fisch ist.

8. Ccbe1 Antisense-Agens, umfassend die Basissequenz von:
5'-CGGGTAGATCATTTCAGACACTCTG-3',
5'-ACAGCACAGCACTTTACCTGTCTAC-3' oder
5'-ATTAGCATAGGGAACTTACTTTCG-3' zur Verwendung in einem Verfahren für die Behandlung von Krebs.

9. Antisense-Agens mit zwischen 15-40 Nukleinbasen in Länge, umfassend mindestens 8 aufeinanderfolgende komplementäre Nukleinbasen, und das in der Lage ist, mit einer Nukleotid-Sequenz, die ccbe1 kodiert, zu hybridisieren, vorzugsweise mit der Basissequenz wie in SEQ ID NR:35 dargestellt, zur Verwendung in einem Verfahren für die Behandlung von Krebs.

10. Verfahren zum Bestimmen, ob eine Person ein Träger von, oder erkrankt ist an, oder Gefahr läuft zu erkranken an einer kongenitalen Lymphgefäßkrankheit, umfassend das Bereitstellen einer Probe von der Person; Bestimmen des Vorhandenseins einer Veränderung in der Sequenz des Ccbe1-Gens und Bestimmen, dass die Person ein Träger ist, oder erkrankt ist an, oder Gefahr läuft zu erkranken an der kongenitalen Lymphgefäßkrankheit, falls die Veränderung anwesend ist.

11. Verfahren nach Anspruch 10, wobei die Lymphgefäßkrankheit ausgewählt ist aus Meige-, Nonne-Milroy- und Hennekam-Syndrom.

12. Verfahren nach Anspruch 10 oder 11, wobei die Veränderung des Ccbe1-Gens in Aminosäurenveränderungen C75S, C102S C174R L229fsX8 und/oder G327R resultiert.

13. Zusammensetzung umfassend ein Ccbe1-Protein oder ein funktionelles Fragment davon, in der Lage ist, eine Lymphgefäßbildung herbeizuführen, oder umfassend eine Ccbe1 codierende Nukleinsäure oder ein funktionelles Fragment davon, in der Lage ist, eine Lymphgefäßbildung herbeizuführen, zur Verwendung als ein Arzneimittel für die Behandlung einer Lymphgefäßkrankheit.

14. Zusammensetzung zur Verwendung nach Anspruch 13, wobei die Lymphgefäßkrankheit eine kongenitale Lymphgefäßkrankheit oder eine erworbene Lymphgefäßkrankheit ist, ausgewählt aus Lymphödem, Krebs und einer Krankheit, die aus dem Entfernen oder dre Bestrahlung von Lymphgefäßen resultiert.

## Revendications

1. Un procédé pour tester si un composé est capable d'inhiber le développement de canaux lymphatiques ou de lymphangiogenèse et/ou de migration de lymphangioblastes chez un animal non humain, un embryon non humain ou une culture cellulaire, comprenant les étapes consistant à:
a. administrer un composé capable d'interagir avec un gène Ccbe1, un transcript de celui-ci ou une protéine de Ccbe1 à un animal non-humain, un embryon non humain ou une culture cellulaire;
b. déterminer si ledit composé inhibe le développement des canaux lymphatiques, la lymphangiogenèse et/ou la migration de lymphangioblastes chez ledit animal non humain, ledit embryon non humain ou ladite culture cellulaire.

2. Un procédé selon la revendication 1, dans lequel le dit composé est choisi dans le groupe consistant en un anticorps dirigé contre la protéine de Ccbe1 ou un fragment fonctionnel en dérivant, et un agent antisens contre Ccbe1.

3. Un procédé pour tester si un composé est capable d'inhiber le développement de canaux lymphatiques ou de la lymphangiogenèse et/ou de la migration de lymphangioblastes chez un animal non humain, un embryon non humain ou une culture cellulaire, comprenant les étapes consistant à:
a. administrer un composé à un animal non humain, un embryon non humain ou une culture cellulaire, ledit animal non-humain, ledit embryon non humain ou la culture de cellules n'exprimant pas la protéine Ccbe1 à un niveau fonctionnel;
b. induire l'expression de protéines Ccbe1 dans des cellules dudit animal non humain ou dudit embryon non humain; et
c. déterminer si ledit composé inhibe le développement des canaux lymphatiques, de la lymphangiogenèse et/ou de la migration de lymphangioblastes dans ledit animal non humain, ledit embryon non humain ou ladite culture cellulaire.

4. Un procédé selon une quelconque des revendications 1 à 3, dans lequel ledit animal ou ledit embryon est transparent.

5. Un procédé selon l'une quelconque des revendications 1 à 4, dans lequel ledit animal ou ledit embryon est génétiquement modifié, où la modification comprend l'expression transgénique de stabilin1, tie2, lyve1 et/ou flil fusionné à une GFP ou un de ses dérivés.

6. Un procédé selon l'une quelconque des revendications 3 à 5, dans lequel ledit animal ou ledit embryon est génétiquement modifié de façon que le gène Ccbe1 soit éliminé.

7. Un procédé selon l'une quelconque des revendications 1 à 6, dans lequel ledit animal ou ledit embryon est un poisson.

8. Un agent anti-sens Ccbe1 comprenant la séquence de base de:
5'-CGGGTAGATCATTTCAGACACTCTG-3',
5'-ACAGCACAGCACTTTACCTGTCTAC-3' ou
5'-ATTAGCATAGGGAACTTACTTTCG-3' destiné à être utilisé dans un procédé de traitement du cancer.

9. Un agent antisens ayant entre 15 à 40 nucléobases de longueur, comprenant au moins huit nucléobases consécutives complémentaires et étant capable de s'hybrider à une séquence nucléotide codant pour Ccbe1, de préférence de la séquence de base telle que représentée dans SEQ ID N° 35 destiné à être utilisé dans un procédé de traitement du cancer.

10. Un procédé pour déterminer si un individu est porteur d'un trouble congénital des canaux lymphatiques, en souffre ou présente le risque d'en souffrir, ce procédé comprenant la fourniture d'un échantillon dudit individu; la détermination de la présence d'une altération dans la séquence du gène Ccbe1, et la détermination que l'individu est porteur d'un trouble congénital des canaux lymphatiques, en souffre ou présente le risque d'en souffrir, si ladite altération est présente.

11. Le procédé selon la revendication 10, dans laquelle ledit trouble des canaux lymphatiques est l'un des troubles consistant en les syndromes de Meige, de Nonne-Milroy ou de Hennekam.

12. Le procédé selon la revendication 10 ou 11, dans lequel l'altération du gène Ccbe1 résulte de changements d'acides aminés de C75S, C102S, C174R, L229fsX8 et/ou G327R.

13. Une composition comprenant la protéine Ccbe1 ou un fragment fonctionnel en dérivant susceptible d'induire la formation de vaisseaux lymphatiques ou comprenant un acide nucléique codant pour Ccbe1 ou un fragment fonctionnel en dérivant susceptible d'induire la formation de vaisseaux lymphatiques, pour son utilisation comme médicament pour le traitement des désordres des vaisseaux lymphatiques.

14. La composition destinée à être utilisée selon la revendication 13, où le désordre des vaisseaux lymphatiques est un désordre congénital des vaisseaux lymphatiques ou un désordre des vaisseaux lymphatiques acquis consistant en lymphodème, cancer et désordres résultant de l'élimination ou de l'irradiation de vaisseaux lymphatiques.
